# EUROPÄISCHE PATENTSCHRIFT

(11) **EP 3 651 805 B1**
(45) Veröffentlichungstag und Bekanntmachung des Hinweises auf die Patenterteilung: **25.10.2023**
(21) Anmeldenummer: 18738321.1
(22) Anmeldetag: 11.07.2018
(51) Int. Cl.: A61K 47/26, A61K 9/107, A61K 31/12, A61P 3/06, A61P 3/04, A61P 3/10, A61K 31/352, A61K 36/185, A61K 36/9066, A61P 1/16, A61K 36/484

(54) **SOLUBILISAT MIT CURCUMIN UND ZUMINDEST EINEM WEITEREN WIRKSTOFF**
SOLUBILISATE WITH CURCUMIN AND AT LEAST ONE OTHER ACTIVE SUBSTANCE
SOLUBILISAT CONTENANT DE LA CURCUMINE ET AU MOINS UNE AUTRE SUBSTANCE ACTIVE

(30) Priorität: 11.07.2017 WO PCT/EP2017/067382; 11.07.2017 WO PCT/EP2017/067381; 11.07.2017 DE 102017115496
(43) Veröffentlichungstag der Anmeldung: 20.05.2020
(73) Patentinhaber: Aquanova AG, 64295 Darmstadt (DE)
(72) Erfinder: BEHNAM, Dariush, 64380 Rossdorf (DE)
(74) Vertreter: Tesch, Sabine
(86) Internationale Anmeldenummer: PCT/EP2018/068731
(87) Internationale Veröffentlichungsnummer: WO 2019/011955

(56) Entgegenhaltungen:
- EP-A1- 0 755 940
- EP-A1- 1 431 385
- WO-A2-2007/006497
- DE-A1-102006 024 911
- DE-U1-202012 012 130
- US-A1- 2011 086 017
- US-A1- 2016 008 298
- US-A1- 2016 081 975
- REJI KIZHAKKEDATH: "Clinical evaluation of a formulation containing Curcuma longa and Boswellia serrata extracts in the management of knee osteoarthritis", MOLECULAR MEDICINE REPORTS, Bd. 8, Nr. 5, 1. November 2013 (2013-11-01), Seiten 1542-1548, XP055462856, GR ISSN: 1791-2997, DOI: 10.3892/mmr.2013.1661
- DATABASE EMBASE [Online] ELSEVIER SCIENCE PUBLISHERS, AMSTERDAM, NL; 1. September 2017 (2017-09-01), KHAYYAL M T: "Novel formulations of Curcumin, Boswellia and Xanthohumol extracts markedly enhance their individual and combined anti-inflammatory activity", XP002783955, Database accession no. EMB-621379886 & ZEITSCHRIFT FUR PHYTOTHERAPIE 20170901 HIPPOKRATES VERLAG GMBH NLD, Bd. 38, Nr. Supplement 1, 1. September 2017 (2017-09-01), ISSN: 1438-9584
- ALEXA KOCHER ET AL: "The oral bioavailability of curcuminoids in healthy humans is markedly enhanced by micellar solubilisation but not further improved by simultaneous ingestion of sesamin, ferulic acid, naringenin and xanthohumol", JOURNAL OF FUNCTIONAL FOODS, Bd. 14, 1. April 2015 (2015-04-01), Seiten 183-191, XP055435032, NL ISSN: 1756-4646, DOI: 10.1016/j.jff.2015.01.045
- ZAMZOW DANIEL R ET AL: "Xanthohumol improved cognitive flexibility in young mice", BEHAVIOURAL BRAIN RESEARCH, Bd. 275, 1. September 2014 (2014-09-01), Seiten 1-10, XP029077816, ISSN: 0166-4328, DOI: 10.1016/J.BBR.2014.08.045
- J Pharm ET AL: "Glycyrrhetinic acid, leucocytes and prostaglandins", J. Pharm. Pharmacol, 1. Januar 1983 (1983-01-01), Seiten 332-335, XP055501119, Gefunden im Internet: URL:https://onlinelibrary.wiley.com/doi/pd f/10.1111/j.2042-7158.1983.tb02949.x
- BHAGAT SHIVANI ET AL: "Serratiopeptidase: A systematic review of the existing evidence", INTERNATIONAL JOURNAL OF SURGERY, SURGICAL ASSOCIATES, LONDON, GB, Bd. 11, Nr. 3, 1. Februar 2013 (2013-02-01), Seiten 209-217, XP028992850, ISSN: 1743-9191, DOI: 10.1016/J.IJSU.2013.01.010
- RAJU GAUTAM ET AL: "Recent developments in anti-inflammatory natural products", MEDICINAL RESEARCH REVIEWS, Bd. 29, Nr. 5, 1. September 2009 (2009-09-01), Seiten 767-820, XP055294630, US ISSN: 0198-6325, DOI: 10.1002/med.20156
- MANJU RAWAT SINGH ET AL: "Development and in vitro evaluation of polar lipid based lipospheres for oral delivery of peptide drugs.", INTERNATIONAL JOURNAL OF DRUG DELIVERY, Bd. 1, Nr. 1, 31. Juli 2009 (2009-07-31), Seiten 15-26, XP055570194, DOI: 10.5138/ijdd.2009.0975.0215.01002
- MANJU RAWAT ET AL: "Formulation optimization of double emulsification method for preparation of enzyme-loaded Eudragit S100 microspheres", JOURNAL OF MICROENCAPSULATION., Bd. 26, Nr. 4, 20. Oktober 2008 (2008-10-20), Seiten 306-314, XP055570200, GB ISSN: 0265-2048, DOI: 10.1080/02652040802319767

## Beschreibung

Die Erfindung betrifft ein Solubilisat mit Curcumin und zumindest einem weiteren Wirkstoff zur Verwendung gemäß Anspruch 1. Des Weiteren betrifft die Erfindung ein Fluid enthaltend ein derartiges Solubilisat, eine Kapsel gefüllt mit einem solchen Solubilisat beziehungsweise Fluid und ein Nahrungsergänzungsmittel und/oder Arzneimittel enthaltend ein solches Solubilisat. Curcumin wird als Wirkstoff basierend auf verschiedenen möglichen pharmakologischen Eigenschaften diskutiert. Beispielsweise gibt es Anhaltspunkte für die antioxidative und auch für die antiinflammatorische Wirkung des Curcumins ebenso wie für die Wirksamkeit gegen Viren und Bakterien sowie gegen Krebs. Indikationen könnten daher beispielsweise Parkinson, Alzheimer, Diabetes, kolorektale Tumoren, Bauchspeicheldrüsenkrebs und Leberfunktionsstörungen sein.

Um nach oraler Einnahme in den Blutkreislauf gelangen zu können, muss der Wirkstoff die Dünndarm-Blutschranke passieren, wird dann in der Leber metabolosiert und gelangt als bioverfügbarer Anteil in die Lebervene. Der Rest des insgesamt eingenommenen und im Körper freigesetzten Wirkstoffes wird entweder mikrobiell im Darm abgebaut oder mit den Fäzes beziehungsweise der Galle eliminiert.

Der Erfinder hat bereits ein Curcuminsolubilisat geschaffen, welches eine gegenüber nativem Curcumin deutlich erhöhte Bioverfügbarkeit aufweist. Dieses Solubilisat wird in der internationalen Patentanmeldung WO 2014094921 A1 beschrieben. Überraschenderweise hat sich in mehreren Untersuchungen herausgestellt, dass dieses Curcuminsolubilisat in seiner spezifischen Formulierung neben der hohen Bioverfügbarkeit auch eine unerwartet größere Wirkung auf die Verringerung von Krankheitssymptomen hat, die insbesondere mit Entzündungen oder Krebs in Verbindung stehen.

Eine Toxizität aufgrund der erfindungsgemäßen Micellierung des Wirkstoffes im Vergleich zur nativen Form konnte anhand von Untersuchungen mit MTT-Assays zur Zelllebensfähigkeit ("viability") ausgeschlossen werden. Der Nachweis der Zellvitalität mittels MTT-Test beruht dabei auf der Reduktion des gelben, wasserlöslichen Farbstoffs 3-(4,5-Dimethylthiazol-2-yl)-2,5-diphenyltetrazoliumbromid (MTT) in ein blau-violettes, wasserunlösliches Formazan.

US 2016/0081975 A1 offenbart nicht wässrige Prä-Gel Konzentrate, die eine wasserlösliche Mischung von Vitamin E Derivaten oder ein Polyethylenglykol(PEG)-Derivat von Vitamin E enthalten, eine von dem PEG-Derivat von Vitamin E verschiedene nicht polare Substanz, und ein nicht wässriges Lösungsmittel. In Tabelle 5 ist ein Prä-Gel Konzentrat mit Curcumin, *Boswellia* Extrakt, Hopfenextrakt und anderen Bestandteilen offenbart.

Der Erfinder stellte sich daher die Aufgabe, eine Formulierung bereitzustellen, welche die gesundheitsfördernden bis heilenden Eigenschaften von Curcumin, auch im Hinblick auf eine Kombination mit zumindest einem weiteren Wirkstoff, dem menschlichen oder tierischen Organismus zugänglich macht. Insbesondere ist es eine Aufgabe der Erfindung, eine möglichst hohe Bioverfügbarkeit von Curcumin in Kombination mit zumindest einem weiteren Wirkstoff zu ermöglichen.

Der Erfinder hat erkannt, dass die positive Wirkung des Curcumins auf Heilungsprozesse beziehungsweise die Gesunderhaltung überraschenderweise in Kombination mit weiteren Wirkstoffen, zumindest teilweise synergistisch, genutzt werden kann.

Die Erfindung ist in den Ansprüchen 1-15 definiert.

Mit dem Begriff "Wirkstoff" wird im Rahmen dieser Anmeldung ein Stoff bezeichnet, der in pharmazeutisch wirksamer Konzentration vorliegt und vorzugsweise zum Zweck einer pharmazeutischen Wirkung beigegeben wird. Dabei sind unter der Bezeichnung des entsprechenden Wirkstoffes auch solche Stoffe zu verstehen, die im Körper in den jeweiligen Wirkstoff und/oder in seine biologisch aktive Form umgewandelt werden.

Zu diesen "Wirkstoffen" im Sinne dieser Anmeldung gehören sekundäre Pflanzenstoffe, welche als chemische Verbindungen von Pflanzen weder im Energiestoffwechsel noch im aufbauenden (anabolen) oder im abbauenden (katabolen) Stoffwechsel produziert werden. Eine Gruppe der sekundären Pflanzenstoffe und damit der Wirkstoffe im Sinne dieser Anmeldung sind die Flavonoide. Auch natürliche Polyphenole wie Resveratrol oder die Polyphenole aus Süßholz und natürliche Phenole, insbesondere Chalkone wie Xanthohumol, gehören zu den "Wirkstoffen" im Sinne dieser Anmeldung. Ebenso zählen Pflanzenextrakte dazu, also Stoffe, die mit Hilfe eines Extraktionsmittels aus Pflanzen oder Pflanzenteilen herausgelöst wurden. Dazu zählen Extrakte aus Hopfen oder aus dem Wurzelmaterial der Süßholzpflanze. Als "Extrakt" wird der Wirkstoff auch dann bezeichnet, wenn er noch im Extraktionsmittel gelöst vorliegt. Auch der Begriff "Auszug" ist für ein Extrakt gebräuchlich.

Zu "Wirkstoffen" im Sinne dieser Anmeldung gehören auch Enzyme. Ein Beispiel für ein Enzym als "Wirkstoff" im Sinn der vorliegenden Anmeldung ist Serrapeptase. Die Anmeldung ist jedoch nicht auf dieses Enzym beschränkt.

Das Extrakt aus dem Harz des Weihrauchbaumes, *Boswellia serrata* Extrakt, enthält mehrere pentazyklische Triterpene, welche zusammen häufig als gesamte Boswelliasäuren ("total BAs") bezeichnet werden. Mit dem Begriff "Boswelliasäuren" wird eine Gruppe chemischer Verbindungen bezeichnet, die natürlich in dem genannten Harz der Weihrauchbäume vorkommen. Die beiden Grundstrukturen sind die α-Boswelliasäure und die β-Boswelliasäure. Von den Boswelliasäuren sind auch einige Derivate bekannt, insbesondere Verbindungen, die an Position 11 eine Ketogruppe tragen und/oder an Position 3 acetyliert sind. Derzeit gelten im Hinblick auf pharmakologische Effekte insbesondere die Boswelliasäuren "αBA" α-Boswelliasäure und "βBA" β-Boswelliasäure sowie deren Derivate "KBA" 11-keto-β-Boswelliasäure (CAS 17019-92-0) und "AKBA" 3-O-Acetyl-11-keto-β-Boswelliasäure (CAS 67416-16-9) sowie "AαBA" 3-O-Acetyl- α-Boswelliasäure und "AβBA" 3-O-Acetyl-β-Boswelliasäure als bedeutungsvoll. Besonders dem Derivat AKBA wird eine entzündungshemmende Wirkung zugeschrieben. Im Rahmen der vorliegenden Anmeldung wird der Ausdruck "Boswellia", insbesondere in dem Begriff "Boswellia-Solubilisat" in dem Sinne gebraucht, dass sich Ausdruck "Boswellia" auf die Wirkstoffe aus dem Harz des Weihrauchbaumes bezieht, also auf zumindest eine Boswelliasäure und/oder zumindest ein Derivat einer Boswelliasäure bezieht. Der Ausdruck "Boswelliasäure-Solubilisat" bezeichnet dabei eine mizellare Formulierung zumindest einer Boswelliasäure. Diese kann auch zumindest ein Boswelliasäurederivat enthalten.

Xanthohumol ist ein natürlich in Hopfen vorkommendes Flavonoid. Dabei handelt sich um ein prenyliertes Pflanzenpolyphenol, das den Chalkonen zugeordnet wird und bisher ausschließlich im Hopfen nachgewiesen werden konnte. Dabei weisen die Bitterhopfensorten einen deutlich höheren Gehalt an Xanthohumol auf als Aromasorten. In Tests zeigte sich Xanthohumol als wirksam gegen die Entstehung und Entwicklung von Krebszellen. In Laborversuchen konnte zudem festgestellt werden, dass Xanthohumol die Nervenzellen des Gehirns schützen kann und dadurch möglicherweise helfen könnte, bei Erkrankungen wie Alzheimer oder Parkinson den Krankheitsverlauf zu verlangsamen.

Süßholzfolavonoid-haltiges Öl ("Licorice flavonoid oil" LFO) bestehend aus hydrophoben Polyphenolen des Süßholzes in mittelkettigen Triglyceriden hat einen gewichtsreduzierenden Effekt, welcher mit reduziertem Körperfett in Verbindung gebracht wird. Ethanolischen Auszügen des Süßholzes werden zudem antioxidative Eigenschaften zugeschrieben. Süßholzfolavonoid-haltiges Öl mit einer Glabridin-Konzentration von 3 % hat den Markennamen "KANEKA GLAVONOID^{™}". Im Folgenden wird sie auch als "Glavonoid" bezeichnet.

Resveratrol ist ein Phytoalexin mit antioxidativen Eigenschaften, das zu den Polyphenolen zählt. Die Substanz findet sich beispielsweise in Weintrauben, in relativ großen Mengen dabei in der Haut von roten Weintrauben, jedoch auch in Himbeeren, Maulbeeren, Pflaumen, Erdnüssen und im Japanischen Staudenknöterich. Auch aus der Weinrebe selbst lässt sich Resveratrol isolieren. Gemäß dem Eintrag in der Online-Enzyklopädie "Wikipedia" haben In-vitro-Studien Hinweise auf eine mögliche Wirksamkeit gegen Krebszellen und positive Effekte bei Krankheiten wie Arteriosklerose, Herzkrankheiten, Alzheimer-Krankheit, Arthritis und manchen Autoimmunkrankheiten erbracht.

Die Serratia Peptidase oder Serrapeptase ist ein proteolytisches Enzym, das durch die Bakterie Serratia, die im Darm der Seidenraupe lebt, produziert wird. Der Serrapeptase werden positive Wirkungen im Zusammenhang der Linderung von Schmerzen, Entzündungen, traumatischen Schwellungen und überschüssigen Schleimausscheidungen durch den Organismus zugeschrieben. Sie soll wie ein Entzündungshemmer und Schmerzstiller in ähnlicher Weise wie Acetylsalicylsäure, Iboprofen oder andere nicht steroidale Schmerzmittel wirken. Sie soll außerdem im Gewebe eine fibrinolytische entzündungshemmende und antiödematöse Aktivität hervorrufen. Wie alle Enzyme ist die Serrapeptase sensibel gegenüber den Säuren, die durch den Magen produziert werden. Daher ist die Bereitstellung in einer Formulierung, die eine Magenpassage ermöglicht, eine Aufgabe der Erfindung.

Das erfindungsgemäße Solubilisat kann eine oder mehrere Boswelliasäuren und/oder eine oder mehrere Boswelliasäurederivate mit einem Anteil von insgesamt kleiner oder gleich 10 Gew.-%, bevorzugt kleiner oder gleich 8 Gew.-%, besonders bevorzugt 4,7 Gew.-% bis 6,6 Gew.-% enthalten.

Aufgrund des hohen Anteils an Boswellia sieht die Erfindung in einer vorteilhaften Ausgestaltung vor, dass das Solubilisat als Quelle dass das Solubilisat als Quelle für die eine oder mehreren Boswelliasäuren und/oder eine oder mehrere Boswelliasäurederivate ein durch Extraktion mittels Essigsäure-Ethylester aus dem Harz der Pflanze *Boswellia serrata* gewonnenes Extrakt enthält, wobei Boswelliasäuren in diesem Auszug in einer Konzentration von mindestens 85 Gew.-% vorliegen.

Das erfindungsgemäße Solubilisat kann Xanthohumol mit einem Anteil von kleiner oder gleich 10 Gew.-%, bevorzugt kleiner oder gleich 5 Gew.-%, besonders bevorzugt 1 Gew.-% bis 3 Gew.-% enthalten.

Aufgrund des hohen Anteils an Xanthohumol sieht die Erfindung in einer vorteilhaften Ausgestaltung vor, dass das Solubilisat als Quelle für Xanthohumol einen ethanolischen Auszug der Hartharze aus Hopfen enthält, wobei Xanthohumol in diesem Auszug in einer Konzentration im Bereich zwischen 65 Gew.-% und 95 Gew.-%, bevorzugt auf eine Konzentration im Bereich von 80 Gew.-% bis 92 Gew.-% vorliegt. Insbesondere die unten näher erläuterten Produkte "Xantho-Flav pur" oder "Xantho-Flav" können im Rahmen der Erfindung als Xanthohumolquelle verwendet werden.

Das erfindungsgemäße Solubilisat kann ein Fluid enthaltend Süßholzwurzelextrakt, insbesondere eine hydrophobe Lösung eines Süßholzwurzelextraktes, bevorzugt Glavonoid, und/oder Glabridin, mit einem Anteil von kleiner oder gleich 35 Gew.-%, bevorzugt kleiner oder gleich 20 Gew.-%, besonders bevorzugt 0,3 Gew.-% bis 17 Gew.-% enthalten.

Das erfindungsgemäße Solubilisat kann Resveratrol im Bereich zwischen 1 Gew.-% und 15 Gew.-%, besonders bevorzugt im Bereich zwischen 5 Gew.-% und 10 Gew.-% enthalten.

Das erfindungsgemäße Solubilisat kann Serrapeptase im Bereich bis 3 Gew.-%, bevorzugt im Bereich zwischen 0,1 Gew.-% und 2 Gew.-%, besonders bevorzugt im Bereich zwischen 0,18 Gew.-% und 0,35 Gew.-% enthalten.

Das erfindungsgemäße Solubilisat kann Coenzym Q10 im Bereich bis 10 Gew.-%, bevorzugt im Bereich zwischen 0,1 Gew.-% und 5 Gew.-%, besonders bevorzugt im Bereich zwischen 0,5 Gew.-% und 1,5 Gew.-% enthalten.

Das erfindungsgemäße Solubilisat kann α-Liponsäure im Bereich bis 10 Gew.-%, bevorzugt im Bereich zwischen 0,1 Gew.-% und 5 Gew.-%, besonders bevorzugt im Bereich zwischen 0,8 Gew.-% und 2,5 Gew.-% enthalten.

Auch ein Solubilisat bestehend aus oder enthaltend Curcumin und zumindest einen weiteren Wirkstoff kann im Rahmen der Erfindung vorteilhafterweise zur Verwendung bei der Vorbeugung vor mit einer Entzündung einhergehenden Krankheiten, Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, zum Senken von Cholesterin, insbesondere LDL-Cholesterin, und/oder von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel zur Behandlung von und/oder Vorbeugung vor Fettleber, Friedreich-Ataxie, lysomale Krankheiten, insbesondere Morbus Tay-Sachs, Arteriosklerose, Herzkrankheiten, Arthritis, vorgesehen sein beziehungsweise eingesetzt werden.

Insbesondere stellt die Erfindung die oben beschriebenen Solubilisate zur Verfügung zur Anwendung als entzündungshemmendes Arzneimittel und/oder als Antibiotikum und/oder als Arzneimittel mit einer Wirkung gegen Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, als cholesterinsenkendes Arzneimittel, insbesondere betreffend LDL-Cholesterin, und/oder als Arzneimittel mit einer Wirkung zur Senkung von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel mit einer Wirkung gegen Fettleber, Friedreich-Ataxie, lysomale Krankheiten, insbesondere Morbus Tay-Sachs, Arteriosklerose, Herzkrankheiten, Arthritis.

Auch in diesem Zusammenhang hat es sich als vorteilhaft erwiesen, dass für das erfindungsgemäße Solubilisat die Gesamt-Curcuminoid-Konzentration im humanen Blutplasma gemessen eine Stunde nach oraler Gabe von 500 mg Curcumin in Form des Solubilisats nach einem der vorangegangenen Ansprüche bei etwa 500 ng Curcuminoid pro mL Plasma ± 100 ng Curcuminoid pro mL Plasma liegt. Die Gesamt-Curcuminoid-Konzentration im humanen Blutplasma über einen Zeitraum von 24 Stunden gemessen als Fläche unter der Kurve der gesamten Curcumin-Konzentration im Blutplasma (area under the total curcumin plasma concentration-time curve AUC) im Bereich von etwa 9.500 bis etwa 10.000 nmol h /L liegt. Damit steht das Solubilisat dem Körper in hohem Masse auch nach oraler Gabe zur Verfügung.

Die Erfindung stellt vorteilhafterweise Solubilisate mit sehr guter entzündungshemmenden Eigenschaften zur Verfügung. Die antiinflamatorische Aktivität gemessen als Konzentration von C-reaktivem Protein (CRP) im Blutserum arthritischer Ratten nach einmaliger Gabe des erfindungsgemäßen Solubilisats in einer Dosierung von 5 mg/kg Körpergewicht Curcumin liegt im Bereich von etwa 2100 pg/mL bis etwa 2500 pg/mL, und nach einmaliger Gabe des Solubilisats in einer Dosierung von 10 mg/kg Körpergewicht Curcumin im Bereich von etwa 1400 pg/mL bis etwa 1800 pg/mL.

Die antiinflamatorische Wirkung gemessen als Konzentration von Myeloperoxidase (MPO) im Blutserum arthritischer Ratten liegt nach einmaliger Gabe des Solubilisats in einer Dosierung von 5 mg/kg Körpergewicht Curcumin im Bereich von etwa 800 mU/mL bis etwa 900 mU/mL. Diese sind deutlich niedriger als die Werte für natives Curcumin, wie unten genauer erläutert wird.

Die antiinflamatorische Aktivität eines erfindungsgemäßen Solubilisats von Curcumin und Boswellia gemessen als Konzentration von C-reaktivem Protein (CRP) im Blutserum arthritischer Ratten liegt nach einmaliger Gabe des Solubilisats in einer Dosierung von 5 mg/kg Körpergewicht Curcumin und 10 mg/kg Körpergewicht Boswelliasäuren im Bereich von etwa 1200 pg/mL bis etwa 1500 pg/mL im Vergleich zu etwa 3200 pg/mL bis etwa 3500 pg/mL nach einer Gabe gleicher Dosierung von nativem Curcumin beziehungsweise Boswellia.

Die antiinflamatorische Wirkung eines erfindungsgemäßen Solubilisats von Curcumin und Boswellia gemessen als Konzentration von Myeloperoxidase (MPO) im Blutserum arthritischer Ratten liegt nach einmaliger Gabe des Solubilisats in einer Dosierung von 5 mg/kg Körpergewicht Curcumin und 10 mg/kg Körpergewicht Boswelliasäuren im Bereich von etwa 750 mU/mL bis etwa 815 mU/mL und damit deutlich niedriger als etwa 1150 mU/mL bis etwa 1250 mU/mL nach einer Gabe gleicher Dosierung von nativem Curcumin beziehungsweise Boswellia.

Die Enzymeinheit (U) ist eine heute mittlerweile durch das Katal abgelöste Einheit zur Angabe der Enzymaktivität. Da sich bei Verwendung des Katal die Zahlenwerte ändern, wird die Enzymeinheit (U) in Medizin und klinischer Chemie weiterhin genutzt. Eine Enzymeinheit U entspricht einem Mikro-Mol Substratumsatz pro Minute.

Des Weiteren bietet die Erfindung den Vorteil, das erfindungsgemäße Solubilisat zur Anwendung im Bereich der Landwirtschaft, Fischzucht und/oder im Gartenbau und/oder im Bereich der Lebensmittelhygiene und/oder zur Anwendung als Desinfektionsmittel und/oder im Bereich der Verpackung, bevorzugt bei der Verpackung von Rindfleisch, Geflügel oder Fisch, und/oder zur Anwendung als Desinfektionsmittel zur Verfügung stellen zu können.

Insbesondere für das Solubilisat alleine mit Curcumin als Wirkstoff konnte gezeigt werden, dass die Keimbelastung von Lebensmitteln durch Behandlung mit dem erfindungsgemäßen Solubilisat zu einer deutlichen Reduzierung der Keimbelastung führt. Das Curcumin wirkt dabei als Photosensibilisator. Dieser wird lokal auf eine keimbefallene Oberfläche aufgebracht und dort von den Keimen aufgenommen, wobei dann die Oberfläche belichtet und dadurch der Photosensibilisator aktiviert wird. Dieser produziert dann reaktive Sauerstoffspezies ("Sauerstoffradikale") in den Mikroben, die dadurch getötet werden.

Im Lebensmittelbereich, beispielsweise bei antimikrobieller Verpackung, kann das Lebensmittel wie etwa Fleisch mit wässriger Lösung eines erfindungsgemäßen Solubilisats besprüht in PP- PE- PC- oder sonstigen Klarsichtfolien verpackt und anschließend belichtet werden, um so die Oberfläche von bereits verpackten Lebensmittel keimfrei zu halten. Entsprechende Anwendungen auch für die keimfreie Verpackung anderer Gegenstände liegen ebenfalls im Rahmen der Erfindung. Dazu gehört beispielsweise die Verpackung von Operationsbesteck und ähnlichen Utensilien.

Der Vorteil der Formulierung als erfindungsgemäßes Solubilisat für diese Art der Anwendung liegt darin, dass der Wirkstoff, insbesondere das Curcumin, in sehr hoher wässriger Verdünnung eingesetzt werden kann, so dass eine Farbbelastung der behandelten Oberfläche im Wesentlichen vollständig ausgeschlossen werden kann. Durch die hohe Anzahl der sehr kleinen Micellen wird auch bei starker Verdünnung des Solubilisats eine gleichmäßige und hinreichende Verteilung des Wirkstoffes auf der zu behandelnden Oberfläche erreicht. Dadurch wird eine Anwendung beispielweise im Bereich möglichst keimfreier Verpackungen in einfacher und sehr kostengünstiger Weise durch die Erfindung ermöglicht.

Um stabile Micellen der Wirkstoffe bereitzustellen muss im Rahmen der Erfindung der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegen.

Je nach Anwendungsfall kann das erfindungsgemäße Solubilisat beispielsweise bis zu 20 Gew.-%, bevorzugt bis zu 15 Gew.-% Ethanol und/oder bis zu 25 Gew.-%, bevorzugt zwischen 12 Gew.-% und 20 Gew.-%, besonders bevorzugt bis zu 10 Gew.-% Glycerin und/oder zusätzlich bis zu 10 Gew.-%, bevorzugt bis zu 7 Gew.-% Wasser enthalten. Durch Zugabe von Ethanol kann der Anteil von Polysorbat reduziert werden, was im Hinblick auf den von WHO empfohlenen ADI-Wert für Polysorbat (25mg/kg-Körpergewicht) einen Vorteil darstellt. Auch durch Zugabe von Glycerin kann der Anteil von Polysorbat reduziert werden.

Die Solubilisate gemäß der Erfindung haben auch unter den physiologischen Bedingungen einer Magenpassage eine enge Partikelgrößenverteilung mit kleinen mittleren Partikelgrößen, bevorzugt reicht die Durchmesserverteilung der Micellen in einer Verdünnung des Solubilisats mit destilliertem Wasser im Verhältnis 1:500 bei pH 1,1 und 37°C von etwa d₁₀=6 nm bis etwa d₉₀=20 nm. Diese Werte wurden anhand einer Volumenverteilung ermittelt. Einzelheiten zur Partikelgrößenanalyse der Mizellen der Solubilisate werden unten erläutert.

Einen Hinweis auf die im Vergleich zu nicht gemäß der Erfindung mizellierten Zusammensetzungen von Curcumin oder von Curcumin und zumindest einem weiteren Wirkstoff verbesserte Bioverfügbarkeit gibt die messtechnisch deutlich einfacher zugängliche Bestimmung der Trübung des Solubilisats. Die Trübung des Solubilisats ist vorzugsweise infolge der erfindungsgemäßen Formulierung kleiner als 25 FNU, bevorzugt kleiner als 3 FNU gemessen durch Streulichtmessung mit Infrarotlicht nach den Vorschriften der Norm ISO 7027 bei einer Verdünnung des Solubilisats im Verhältnis 1:50 oder 1:500 in Wasser bei physiologischen Bedingungen (pH 1,1 und 37°C).

Um die orale Anwendung des erfindungsgemäßen Solubilisats in für den Konsumenten beziehungsweise Patienten einfacher und angenehmer Weise zu ermöglichen, stellt die Erfindung zudem eine Kapsel gefüllt mit einem oben beschriebenen Solubilisat zur Verfügung, wobei die Kapsel als Weichgelatinekapsel oder Hartgelatinekapsel oder als weiche, gelatinefreie Kapsel oder als harte, gelatinefreie Kapsel wie z.B. Cellulose-Kapsel ausgebildet ist.

Das erfindungsgemäße Solubilisat kann im Rahmen der Erfindung zudem in andere Fluide, insbesondere Flüssigkeiten eingearbeitet werden. Dabei bleiben die wirkstoffgefüllten kleinen Mizellen erhalten. Somit stellt die Erfindung auch ein Fluid enthaltend oben beschriebenes Solubilisat zur Verfügung, wobei das Fluid aus der Gruppe ausgewählt ist, welche Lebensmittel, Nahrungsergänzungsmittel, Getränke, Kosmetika und pharmazeutische Produkte umfasst. Insbesondere kann das Fluid im Rahmen der Erfindung eine wässrige Verdünnung des Solubilisats umfassen.

In einer bevorzugten Ausführungsform des erfindungsgemäßen Solubilisats ist das Solubilisat zur Anwendung als Nahrungsergänzungsmittel und/oder Arzneimittel in einer Curcumin-Dosis im Bereich von 0,5 mg/kg Körpergewicht bis 1 mg/ kg Körpergewicht, bevorzugt mit einer Dosis von 0,81 mg/kg Körpergewicht ausgebildet.

In einer bevorzugten Ausführungsform des erfindungsgemäßen Solubilisats ist das Solubilisat zur Anwendung als Nahrungsergänzungsmittel und/oder Arzneimittel in einer Boswellia-Dosis im Bereich von 1 mg/kg Körpergewicht bis 2 mg/ kg Körpergewicht, bevorzugt mit einer Dosis von 1,62 mg/kg Körpergewicht, ausgebildet.

In einer bevorzugten Ausführungsform des erfindungsgemäßen Solubilisats ist das Solubilisat zur Anwendung als Nahrungsergänzungsmittel und/oder Arzneimittel in einer Xanthohumol-Dosis im Bereich von 0,5 mg/kg Körpergewicht bis 1 mg/kg Körpergewicht, bevorzugt mit einer Dosis von 0,81 mg/kg Körpergewicht, ausgebildet.

Zur Herstellung eines erfindungsgemäßen Solubilisats mit Curcumin und zumindest einem weiteren Wirkstoff können entweder einzeln hergestellte Solubilisate miteinander gemischt werden oder ein Solubilisat enthaltend Curcumin und zumindest einen weiteren Wirkstoff wird direkt hergestellt.

Die Erfindung stellt des Weiteren Verfahren zum Herstellen eines oben beschriebenen Solubilisats bereit. Wird eine Co-Mizellierung von Curcumin und zumindest einen weiteren Wirkstoff angestrebt, stellt die Erfindung folgende erste Variante für ein Herstellungsverfahren bereit mit den Schritten
a) Vorlegen von Polysorbat 80 und/oder Polysorbat 20 und/oder eine Mischung aus Polysorbat 20 und Polysorbat 80
b) Zugabe zumindest eines weiteren Wirkstoffes, insbesondere *Boswellia* serrata-Extrakt und/oder Xanthohumol,
c) Zugabe von Curcumin Pulver
wobei in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt und wobei in Schritt b) die Temperatur unverändert bleibt gegenüber Schritt a) oder eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt
und wobei in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

Durch diese Herstellungsweise wird es ermöglicht, ein Solubilisat zu produzieren, welches in wässriger Verdünnung Mizellen ausbilden kann, welche sowohl mit Curcumin als auch mit zumindest einem weiteren Wirkstoff beladen sind. Dazu können die zumindest zwei Wirkstoffe auch bei einer entsprechend angepassten Temperaturführung in einem vorbereitenden Schritt miteinander gemischt und dann als Mischung gemeinsam zugegeben werden.

Insbesondere kann dabei vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt werden.

Zusätzlich oder alternativ kann in Schritt b1) auch die Zugabe von Ethanol bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt werden.

Eine weitere Möglichkeit für ein Herstellungsverfahren mit einer Co-Micellierung von Curcumin und zumindest einem weiteren Wirkstoff stellt die Erfindung mit folgender zweiter Variante bereit mit einem Verfahren mit den Schritten
a) Herstellen eines ersten Ansatzes durch Vorlegen von Polysorbat 80 und/oder Polysorbat 20 und/oder eine Mischung aus Polysorbat 20 und Polysorbat 80 und von mindestens einem ersten Wirkstoff, insbesondere α-Liponsäure,
b) Herstellen eines zweiten Ansatzes durch Vorlegen von Wasser und zumindest einem weiteren Wirkstoff, insbesondere Serrapeptase
c) Zugabe von zumindest einem weiteren Wirkstoff, insbesondere Xanthohumol und/oder, Boswellia serrata-Extrakt und/oder Glavonoid und/oder Resveratrol und/oder Coenzym Q10, und Zugabe von Curcuminpulver zu dem zweiten Ansatz aus Schritt b)
d) Vereinigen des ersten Ansatzes aus Schritt a) und des zweiten Ansatzes aus Schritt c),
   wobei die Temperatur während der Durchführung der Schritte a) bis d) im Bereich von 18°C bis 22°C liegt,
e) Erwärmung auf eine Temperatur im Bereich von 80°C bis 97°C, bevorzugt auf eine Temperatur im Bereich von 83°C bis 92°C, besonders bevorzugt auf eine Temperatur im Bereich von 85°C und 89°C.

In Schritt c) kann dabei auch die Zugabe von Ethanol und/oder Glycerin und/oder MCT-Öl und/oder Polysorbat 20 und/oder Polysorbat 80 und/oder einer Mischung von Polysorbat 20 und Polysorbat 80 erfolgen.

Eine weitere Möglichkeit für ein Herstellungsverfahren mit einer Co-Micellierung von Curcumin und zumindest einem weiteren Wirkstoff stellt die Erfindung mit folgender dritter Variante bereit mit einem Verfahren mit den Schritten
a) Vorlegen von Polysorbat 80 und/oder Polysorbat 20 und/oder eine Mischung aus Polysorbat 20 und Polysorbat 80 und von Glycerin und von Ethanol,
b) Zugabe zumindest eines weiteren Wirkstoffes, insbesondere eines ethanolischen Auszugs der Hartharze aus Hopfen, insbesondere Xantho-Flav pur-Pulver,
   wobei in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt und wobei in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt,
c) Zugabe von Curcuminpulver bei einer Temperatur im Bereich von 70°C bis 92°C, bevorzugt bei einer Temperatur im Bereich von 75°C bis 87°C, besonders bevorzugt bei einer Temperatur im Bereich von 78°C und 82°C,
d) Zugabe von Glavonoid unter Erwärmung auf eine Temperatur im Bereich von 80°C bis 97°C, bevorzugt auf eine Temperatur im Bereich von 83°C bis 92°C, besonders bevorzugt auf eine Temperatur im Bereich von 85°C und 89°C.

Die Erfindung stellt ebenso ein Verfahren zum Herstellen eines oben beschriebenen Solubilisats durch Mischen eines Curcuminsolubilisats und eines Solubilisats zumindest eines anderen Wirkstoffes, insbesondere im Mengenverhältnis 1:1, zur Verfügung. Dazu stellt die Erfindung auch Solubilisate bereit, welche unmittelbar nach ihrer Herstellung sowohl allein mit Curcumin als auch allein mit jeweils einem anderen Wirkstoff beladene Mizellen in wässriger Verdünnung zeigen..

Die Erfindung wird nachfolgend anhand von Ausführungsbeispielen näher erläutert. Dabei wurden die folgenden Komponenten verwendet.

### Curcumin

Als Curcumin würde das Produkt mit dem Namen "Turmeric Oleoresin Curcumin Powder 95 %" mit dem Produktcode EP-5001 der Green Leaf Extractions Pvt Limited, Kerala, Indien, verwendet. Das Curcuminpulver trägt die CAS-Nr. 458-37-7. Es handelt sich um ein Naturprodukt, welches durch Lösungsmittelextraktion der Rhizome von *Curcuma Longa* gewonnen wird. Der Curcumin-Gehalt des Pulvers beträgt nach Herstellerangaben mindestens 95 %. Dieser Curcumin-Gehalt wird durch die ASTA-Methode 18.0 bestimmt.

In den unten beschriebenen Ausführungsbeispielen können alternativ zu dem erwähnten "oleoresin turmeric 95%"-Curcuminpulver von Greenleaf als Curcumin beispielsweise auch 95%iges Curcuminextrakt von Neelam Phyto-extracts, Mumbai, Indien oder Curcumin BCM-95-SG beziehungsweise Curcumin BCM-95-CG der eurochem GmbH, Gröbenzell, Deutschland oder Curcuma Oleoresin 95 % der Henry Lamotte OILS GmbH, Bremen, Deutschland verwendet werden.

### Boswellia

Mit dem Begriff "Boswellia" wird im Rahmen der vorliegenden Anmeldung insbesondere ein Extrakt aus dem Harz der Weihrauchpflanze bezeichnet. Speziell wurde ein Extrakt der Art *boswellia serrata* verwendet. Dabei handelte es sich um ein durch Extraktion mittels Essigsäure-Ethylester aus dem Harz der Pflanze mit dem botanischen Namen *boswellia serrata* gewonnenes Extrakt mit dem Produktcode "HC22519" des Herstellers Frutarom Belgium N.V., Londerzeel, Belgien. Ein Solubilisat enthaltend diesen Extrakt wird wegen dessen Gehalts an Boswelliasäuren auch als "Boswelliasäuren-Solubilisat" bezeichnet.

Neben Extrakten aus dem Harz der Weihrauchpflanze können für den Zweck der erfindungsgemäßen Solubilisate auch Boswelliasäuren und/oder Derivate der Boswelliasäuren verwendet werden. Dabei kommen insbesondere die Alpha-Boswelliasäure (CAS-Nr. 471-66-9), die Beta-Boswelliasäure (CAS-Nr. 631-69-6) sowie deren Derivate 3-O-Acetyl-Alpha-Boswelliasäure (CAS-Nr. 89913-60-0), 3-O-Acetyl-Beta-Boswelliasäure (CAS-Nr. 5968-70-7), 11-Keto-Beta-Boswelliasäure (KBA, CAS-Nr. 17019-92-0) und 3-O-Acetyl-11-Keto-Beta-Boswelliasäure (AKBA, CAS-Nr. 67416-61-9) in Frage.

### Xanthohumol

Als Xanthohumol-Quelle wurden die Produkte "Xantho-Flav" oder "Xantho-Flav pur" der Marke "Hopsteiner" der Simon H. Steiner, Hopfen, GmbH, Mainburg, Deutschland verwendet. Bei beiden handelt es sich um ein Naturprodukt, das aus Hopfen hergestellt wird. Der aktive Inhaltsstoff ist das Hopfen-Polyphenol Xanthohumol. Es handelt sich um ein gelbfarbenes Pulver mit einem Xanthohumol-Gehalt nach Herstellerangaben zwischen 65 % und 85 % in "Xantho-Flav" und von mindestens 85 % bei "Xantho-Flav pur".

Die Konzentrationen an Xanthohumol und Isoxanthohumol in "Xantho-Flav pur" werden vom Hersteller nach der UVspektrophotometrischen Analyse oder nach HPLC EBC 7.8 über externen Kalibrierstandard reines XN (370 nm) bzw. IX (290 nm) quantifiziert. "Xantho-Flav pur" enthält das prenylierte Flavonoid Xanthohumol in sehr hoher Konzentration. Für die Ausführungsbeispiele im Rahmen der vorliegenden Anmeldung wurde "Xantho-Flav pur" der Batchnummer 9432 verwendet.

### Glavonoid / Glabridin

"Glavonoid" ist der Produktname für eine Zusammensetzung der Kaneka Corporation, Osaka, Japan, welche als Wirkstoff Glabridin enthält. Glabridin ist ein Flavonoid des Echten Süßholzes (*Glycyrrhiza glabra*). Das Produkt "Kaneka Glavonoid" enthält nach Herstellerangaben 30 % Süßholzextrakt und 70 % Speiseöl. "Kaneka Glavonoid" ist nach Herstellerangaben auf 3 % Glabridin standardisiert, welches die Hauptkomponente der Polyphenole des Echten Süßholzes ist. Die CAS-Nr. von Glabridin ist 59870-68-7.

### Resveratrol

Als Resveratrol wurde das Produkt "eveResveratrol" eingesetzt. Es handelt sich dabei um Transresveratrol, das durch Fermentation gewonnen wurde. Das Produkt wird als weißliches Pulver ohne Zusatz von Hilfsstoffen bereitgestellt. Der Anteil an Transresveratrol liegt bei mindestens 98 Gew.-%, der Rest ist Wasser. Das Produkt hat die CAS-Nummer 501-36-0 und den Produktcode RSXOL 9800.

### Serrapeptase

Als Serrapeptase wurde das Produkt mit der Bezeichnung Serratiopeptidase der Shaanxi Pionieer Biotech Co. Ltd mit der Batch-Nummer PBD 20170708 verwendet. Es handelt sich um ein gräulich weißes bis hellbraunes Pulver.

### Coenzym Q10

Co-Enzym Q10 wurde von der Xiamen Kingdomway Group Company bezogen. Es wurde durch mikrobielle Fermentation produziert und enthält nach Herstellerangaben weniger als 0,5 % des Cis-Isomers.

### α-Liponsäure ("alpha"-Liponsäure)

Alpha Liponsäure wurde von der Jiangsu Tohope Pharmaceutical Co. Ltd, China, bezogen.

### Polysorbat 80

Als Quelle für Polysorbat 80 wurde das Material "TEGO SMO 80 V FOOD" mit dem Spezifikationscode "K04 EU-FOOD" der Evonik Nutrition & Care GmbH, Essen, Deutschland, verwendet. Das Produkt entspricht den EU-Anforderungen des Lebensmittelzusatzstoffes E 433. In den unten beschriebenen Ausführungsbeispielen können alternativ zu dem erwähnten TEGO SMO 80 V von Evonik als Polysorbat 80 auch TEGO SMO 80 V von der InCoPA Gmbh, Illertissen, Deutschland oder Crillet 4/Tween 80-LQ-(SG) von der CRODA GmbH, Nettetal, Deutschland oder Lamesorb SMO 20 sowie Kotilen-O/1 VL von Univar oder der Kolb Distributions AG, Hedingen, Schweiz verwendet werden.

### Polysorbat 20

Als Quelle für Polysorbat 20 wurde das Material "TEGO SML 20 V FOOD" mit dem Spezifikationscode "K09 EU-FOOD" der Evonik Nutrition & Care GmbH, Essen, Deutschland, verwendet. Das Produkt entspricht den EU-Anforderungen des Lebensmittelzusatzstoffes E 432. Alternativ zu dem erwähnten TEGO SML 20 von Evonik kann im Rahmen der Erfindung als Polysorbat 20 auch Crillet 1/Tween 20-LQ-(SG) von der CRODA GmbH, Nettetal, Deutschland verwendet werden.

### Ethanol

Ethanol wurde im Rahmen der vorliegenden Anmeldung von der Berkel pfälzische Spritfabrik GmbH & Co. KG bezogen. Gemäß der Spezifikation für "Neutralalkohol unvergällt" 1411U versteuert" beträgt der Gehalt an Ethanol dieses Produkts etwa 92,6 bis 95,2 Gewichts-%.

### Glycerin

Als Glycerin wurde im Rahmen der vorliegenden Anmeldung das Produkt "Glycamed 99,7 %" der Glaconchemie GmbH, Merseburg, Deutschland, verwendet. Nach Herstellerangaben liegt der Glycerin-Gehalt dieses Produkts bei mindestens 99,5 %.

### Mittelkettige Triglyceride

Mittelkettige Triglyceride, engl. medium-chain triglycerides (MCTs) sind Triglyceride, die mittelkettige Fettsäuren enthalten. Zu den mittelkettigen Fettsäuren zählen die Capronsäure, Caprylsäure, Caprinsäure und die Laurinsäure. Es handelt sich dabei um gesättigte Fettsäuren, welche natürlich in tropischen Pflanzenfetten wie Kokosfett und Palmkernöl vorkommen. Zu einem geringen Teil sind sie auch im Milchfett enthalten. In der Natur gibt es kein reines MCT-Öl, synthetisch lassen sich jedoch reine MCT-Öle gewinnen. Im Rahmen der Erfindung können als mittelkettige Triglyceride einzelne MCTs oder eine Mischung unterschiedlicher MCTs eingesetzt werden. Mittelkettige Triglyceride wurden als MCT-Öl Delios VK koscher des Herstellers Cognis GmbH, Monheim, Deutschland, oder als MCT-Öl (70/30) Rofetan GTCC 70/30 des Herstellers DHW Deutsche Hydrierwerke Rodleben GmbH, Dessau-Roßlau, Deutschland, CAS-Nummer 73-398-61-5 eingesetzt.

Des Weiteren können mittelkettige Triglyceride in Form des Produktes ROFETAN DTCC 70/30 (Ph. Eur.) verwendet werden. Dabei handelt es sich um ein Capryl/Caprinsäuretriglycerid mit der CAS-Nummer 73398-61-5. Das Produkt entspricht der Monografie "mittelkettige Triglyceride" des zum Anmeldetag gültigen Europäischen Arzneibuches. Hersteller sind die Ecogreen Oleochemicals DHW, Deutsche Hydrierwerke GmbH, Rodleben, Deutschland.

### Mischtocopherol

Als Mischtocopherol (E306, CAS-Nummern 59-02-9, 16698-35-4, 54-28-4 und 119-13-1) kann beispielsweise das 70%ige Mischtocopherol in Pflanzenöl Vitapherole T-70 Non GMO des Herstellers VitaeNaturals eingesetzt werden.

Wird bei der Herstellung eines Solubilisats zugegeben, wird destilliertes Wasser verwendet.

An der Universität Kairo in der Fakultät für Pharmazie am Institut für Pharmakologie wurden von Herrn Prof. Dr. M. T. Khayyal Untersuchungen zum anti-inflammatorischen Effekt von Curcumin und Kombinationen aus Curcumin mit Boswellia oder Xanthohumol jeweils in nativer oder in gemäß der Erfindung solubilisierter Form durchgeführt.

Es wurden anti-inflammatorische Marker und die antioxidative Kapazität bestimmt. Weibliche Wistar-Ratten mit einem Körpergewicht zwischen 150 und 200 g wurden gemäß "Pearson et al. (1956)" der "Adjuvant induced arthritis" ausgesetzt. Den Tieren wurde über eine subplantare Injektion 0,1 ml Freund's Adjuvans (FCA) am Tag 0 in die rechte Hinterpfote verabreicht. Die Tiere wurden nach dem Zufallsprinzip in 12 Gruppen mit jeweils 8 Tieren aufgeteilt.
Gruppe 1 bildete die Kontrollgruppe.
Gruppe 2 erhielt Diclofenac als Referenzwirkstoff in einer Dosierung von 3 mg/kg Körpergewicht.
Gruppe 3 erhielt natives Curcumin in einer Dosierung von 5 mg/kg Körpergewicht.
Gruppe 4 erhielt gemäß der Erfindung solubilisiertes Curcumin in einer Dosierung von 5 mg/kg Körpergewicht.
Gruppe 5 erhielt natives Curcumin in einer Dosierung von 10 mg/kg Körpergewicht und
Gruppe 6 in derselben Dosierung solubilisiertes Curcumin.
Gruppe 7 erhielt natives Xanthohumol in einer Dosierung von 5 m/kg Körpergewicht und
Gruppe 8 solubilisiertes Xanthohumol in derselben Dosierung.
Gruppe 9 erhielt eine Mischung aus nativem Curcumin in einer Dosierung von 5 mg/kg Körpergewicht und nativem Boswelliaextrakt in einer Dosierung von 10 mg/kg Körpergewicht.
Gruppe 10 erhielt eine Mischung von solubilisiertem Curcumin und solubilisiertem Boswellia in jeweils derselben Dosierung.
Gruppe 11 erhielt eine Mischung aus nativem Curcumin in einer Dosierung von 5 mg/kg Körpergewicht und nativem Xanthohumol in einer Dosierung von 5 mg/kg Körpergewicht und
Gruppe 12 erhielt eine Mischung aus solubilisiertem Curcumin und solubilisiertem Xanthohumol in jeweils derselben Dosierung.

Alle Extrakte bzw. Solubilisate wurden einmal täglich von Tag 0 bis Tag 21 nach der Impfung mit dem Adjuvans oral verabreicht. Nach Tag 21 wurden die Tiere getötet und Serumproben präpariert und bei - 80 °C gelagert. Gemessen wurden Myeloperoxidase (MPO), C-reaktives Protein (CRP), die gesamte antioxidative Kapazität (TAC) und die (thiobarbituratic acid reactive substances) (TBARS).

Die Ergebnisse werden im Folgenden anhand er beigefügten Figuren erläutert. Es zeigen
- Figur 1a: Effekt von Curcumin in nativer und solubilisierter Form und von Diclofenac auf den Serumgehalt an CRP (pg/L),
- Figur 1b: Effekt von Curcumin (5mg/mL) in nativer und solubilisierter Form gegeben zusammen mit entweder Boswellia oder Xanthohumol im Vergleich zu Diclofenac auf den Serumgehalt an CRP (pg/L),
- Figur 2: Effekt von Curcumin in nativer und solubilisierter Form und von Diclofenac auf den Serumgehalt an MPO (mU/mL),

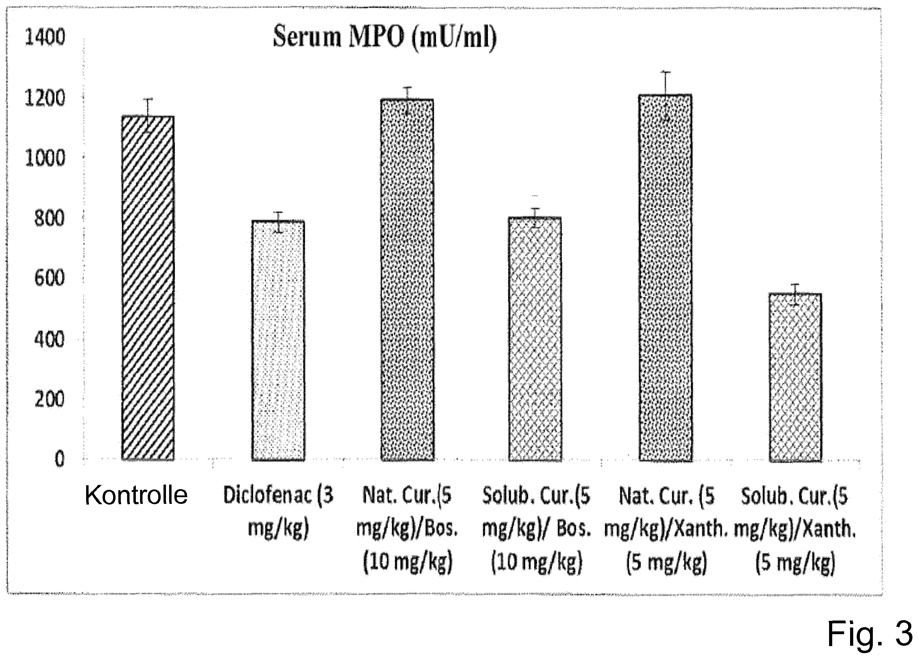

- und Figur 3: Effekt von Curcumin in nativer und solubilisierter Form gegeben zusammen mit entweder Boswellia oder Xanthohumol im Vergleich zu Diclofenac auf den Serumgehalt an MPO (mU/mL).

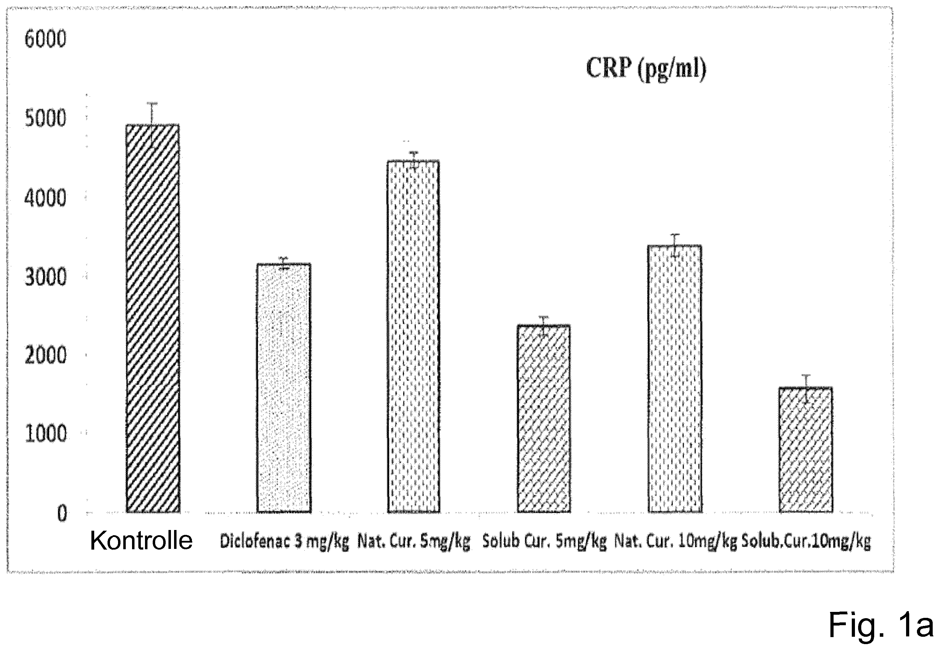

Zunächst wurden die Auswirkungen auf das C-reaktive Protein (CRP) untersucht. C-reaktives Protein ist ein spezifischer Marker für eine antiinflammatorische Aktivität. Sowohl die native als auch die solubilisierte Form von Curcumin hemmte das CRP-Serumlevel der Ratten in dosisabhängiger Weise, wobei die solubilisierte Form jedoch doppelt so wirksam war wie die native und deutlich wirksamer als Diclofenac in der gewählten Dosis (Figur 1a).

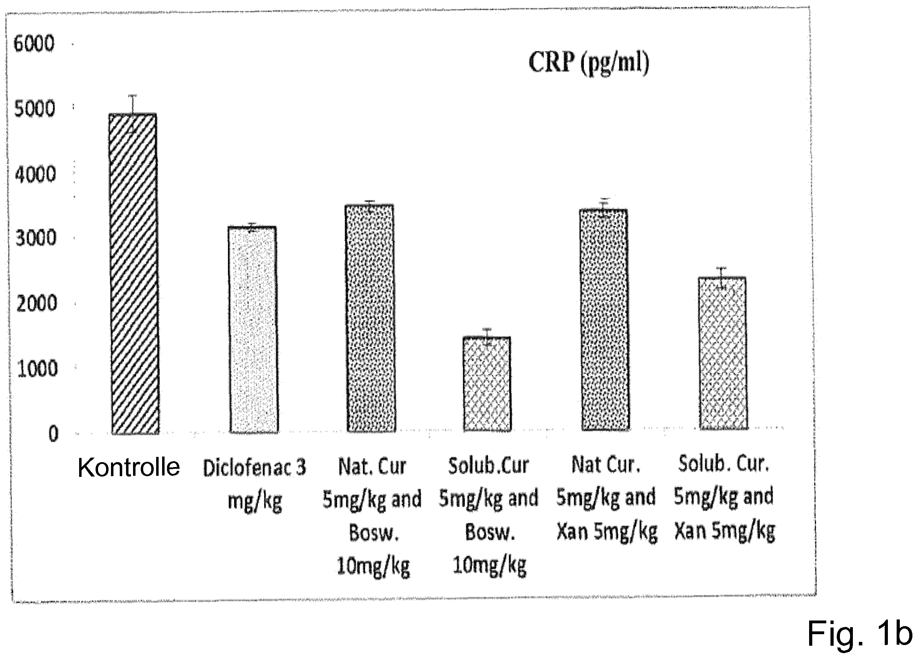

Wird Boswellia gemeinsam mit Curcumin in jeweils nativer Form verabreicht, ändert sich der inhibitorische Effekt auf das Serum CRP nicht signifikant (Figur 1b). Wenn jedoch gemeinsam und in entsprechender Dosierung Curcumin und Boswellia in solubilisierter Form verabreicht werden, wird der antiinflammatorische Effekt verstärkt. In dieser Beziehung ist Boswellia Xanthohumol darin überlegen, den Effekt von Curcumin bei den betrachteten Dosierungen zu potenzieren.

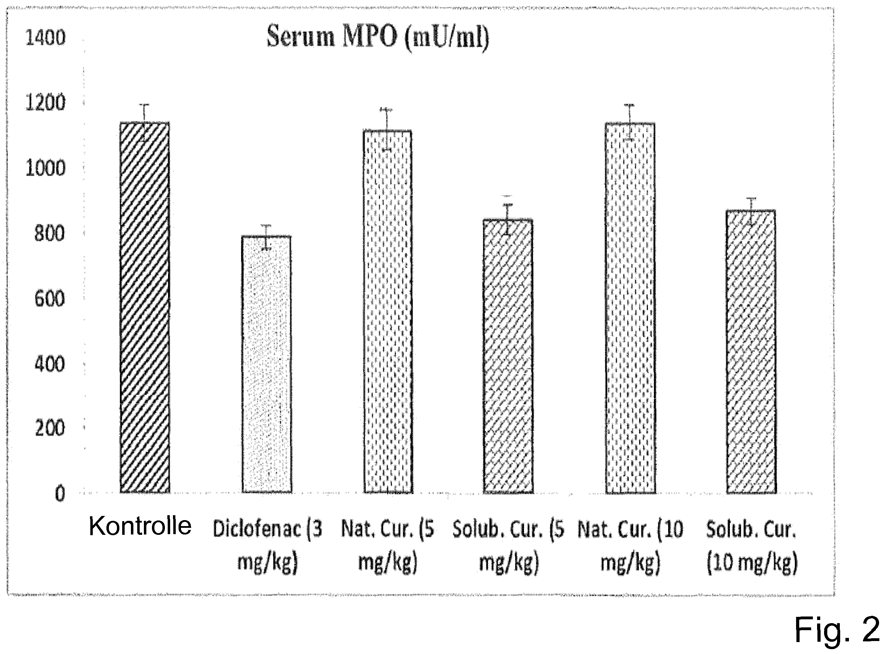

Myeloperoxidase (MPO) im Plasma spielt eine zentrale Rolle als pro-inflammatorischer Mediator in rheumatoider Arthritis und ist ein Indikator für das Einwandern neutrophiler Granulozyten in das betroffene Gewebe. Seine Konzentration ist bei Patienten mit rheumatoider Arthritis erhöht und verursacht oxidativen Stress. In den Untersuchungen wurde die Konzentration an MPO durch solubilisiertes Curcumin effizient reduziert, und zwar bei beiden betrachteten Dosierungen in gleicher Weise und nicht signifikant unterschiedlich von Diclofenac. Allerdings beeinflusste natives Curcumin die MPO-Level nicht (Figur 2). Die Gabe von Boswellia zusammen mit Curcumin sowohl in nativer als auch in solubilisierter Form verbesserte den Effekt des Curcumins bei der Reduzierung der MPO-Konzentration nicht.

Oxidativer Stress ist einer der Hauptfaktoren, die bei rheumatoider Arthritis (RA) zur Gelenkzerstörung beitragen. Ein Anstieg der Produktion von sogenannten "reactive oxigen species (ROS)" führt zu einer verminderten Zufuhr von endogenen Antioxidantien und resultiert schließlich in der Zerstörung von Zellen. Die neutrophilen Granulozyten, welche im rheumatoiden Gelenk freigesetzt werden, produzieren freie Sauerstoffradikale, die zu einer erhöhten Bildung von Lipidperoxiden führen, welche sich in einem Anstieg im Serum TBARS zeigen. Daher kann der Anstieg im Antioxidantien-Status, welcher durch eine Zunahme im TAC repräsentiert wird, als Anzeige des Schutzes gegen die Entwicklung degenerativer entzündlicher Prozesse genutzt werden. Es gibt eine inverse Beziehung zwischen dem Level des TAC und dem des TBARS, ein hohes Level der antioxidativen Kapazität TAC korrespondiert zu einer geringen Konzentration von TBARS.

Die durchgeführten Untersuchungen zeigten, dass die native Form von Curcumin bei beiden betrachteten Dosierungen keinen signifikanten Einfluss auf die Level von TBARS oder TAC hat. Solubilisiertes Curcumin gemäß der Erfindung reduzierte bei beiden ausgewählten Dosierungen den Level von TBARS und erhöhte die TAC, wobei kaum Unterschiede zur Wirkung von Diclofenac erkennbar sind.

Diese Daten sind in der folgenden Tabelle zusammengestellt. Die Tabelle enthält Daten zum Effekt von Curcumin und Boswellia in nativer und solubilisierter Form entweder alleine gegeben oder in Kombination mit Diclofenac in einer Dosierung von 3 mg/kg Körpergewicht einmal täglich über 21 Tage auf die antioxidative Kapazität TAC und die thiobarbitursäure-reaktiven Substanzen TBARS im Serum arthritischer Ratten (n=8). Angegeben sind Mittelwerte ± Standardfehler SEM.

| Gruppe | TAC (nmol/Mikroliter) | TBARS (nmol/L) |
|---|---|---|
| Arthritische Kontrollgruppe | 57,26 ± 3,36 | 13,10 ± 0,39 |
| Diclofenac (3 mg/kg) | 82,08 ± 2,96 | 7,93 ± 0,84 |
| Natives Curcumin (5 mg/kg) | 60,31 ± 3,25 | 11,64 ± 0,39 |
| Solubilisiertes Curcumin (5 mg/kg) | 77,01 ± 0,73 | 5,97 ± 0,47 |
| Natives Curcumin (10 mg/kg) | 68,41 ± 1,09 | 13,18 ± 0,46 |
| Solubilisiertes Curcumin (10 mg/kg) | 87,15 ± 5,27 | 6, 82 ± 0,56 |
| Natives Curcumin (5 mg/kg) + Boswellia (10 mg/kg) | 64,56 ± 1,45 | 12,08 ± 0,52 |
| Solubilisiertes Curcumin (5 mg/kg) + Boswellia (10 mg/kg) | 76, 94 ± 2,17 | 6,81 ± 0,19 |

Das Verabreichen von Boswellia zusammen mit Curcumin in jeweils nativer Form zeigte gemäß den in der Tabelle dargestellten Ergebnissen keinen signifikanten Effekt zur Reduzierung des oxidativen Stresses. In solubilisierter Form waren diese Kombinationen jedoch genauso wirksam wie Diclofenac bei der Reduzierung von TBARS und der Erhöhung des TAC im Serum der arthritischen Ratten.

Erste Untersuchungen zur Anwendung des erfindungsgemäßen Curcuminsolubilisats bei der Bekämpfung von Krebszellen der Linien (MCF-7) und (T47D) für Brustkrebs, (Hepg-2) für Leberkrebs, (HCT-116) für Darmkrebs und (PC3) für Prostatakrebs zeigen sehr gute Ergebnisse im Hinblick auf das Erreichen eines möglichst geringen Anteils an Zellen, die die Behandlung überleben. Das Curcuminsolubilisat erlaubte dabei eine Reduzierung auf eine "surviving fraction" im Bereich von etwa 15 % bis etwa 25 %.

Die Partikelgrößenanalysen der Micellen in wässrigen Verdünnungen erfindungsgemäßer Solubilisate wurden, soweit nicht anders angegeben, gemessen nach dem Prinzip der dynamischen Lichtstreuung mit Laserlicht der Wellenlänge 780 nm. Die Partikelgrößenmessungen wurden mit dem ParticleMetrix NANOFLEX Rückstreu-Teilchenanalysator durchgeführt. Das Messprinzip beruht auf der dynamischen Lichtstreuung (DLS) in einer 180° Heterodyn-Rückstreuanordnung.

Zur experimentellen Bestimmung der Trübung der erfindungsgemäßen Solubilisate werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometric Units". Dies ist die beispielsweise in der Wasseraufbereitung verwendete Einheit für die Messung bei 90° gemäss den Vorschriften der Norm ISO 7072.

Zur Herstellung eines erfindungsgemäßen Solubilisats mit den Wirkstoffen Curcumin und zumindest einem weiteren Wirkstoff können entweder einzeln hergestellte Solubilisate miteinander gemischt werden oder ein Solubilisat enthaltend Curcumin und zumindest einen anderen Wirkstoff oder mehrere weitere Wirkstoffe wird direkt hergestellt.

### Curcuminsolubilisate

Beispielhaft wird ein 7 %iges Curcumin-Solubilisat hergestellt. Dazu werden

| | |
|---|---|
| 925 g | Polysorbat 80 |
| 75 g | Curcuminpulver 95 % (= 71,2 g Curcumin) |

verwendet. Das Polysorbat 80 wird auf 48 bis 52°C erwärmt. Unter Rühren wird das Curcumin-Pulver zum Polysorbat gegeben und dabei weiter auf eine Temperatur im Bereich von 95 bis 97°C erwärmt. Die Zugabe des Pulvers erfolgt mit einer solchen Geschwindigkeit, dass es beim Rühren gleichmäßig in den Emulgator eingezogen wird. Nach Abkühlen auf eine Temperatur unterhalb von maximal 60°C wird das Curcumin-Solubilisat abgefüllt. Dieses Solubilisat wurde für die Herstellung eines Curcumin- und Boswellia-Solubilisats verwendet.

Bei einer Verdünnung im Verhältnis 1:500 in Wasser bei einem pH-Wert von 1,1 und einer Temperatur von 37°C hat das 7 %ige Curcumin-Solubilisat eine gemittelte Trübung von 0,9 FNU.

Es sei jedoch angemerkt, dass der Curcumin-Gehalt sich weiter erhöhen lässt, ohne damit negative Folgen, beispielsweise für die Stabilität der Mizellen in Kauf nehmen zu müssen. Eine Zusammensetzung aus 100 g Curcumin-Pulver 95 %ig und 900 g Polysorbat 80 führt genauso zu einem stabilen Produkt wie eine Zusammensetzung aus 120 g Curcumin-Pulver 95 %ig und 880 Polysorbat 80 oder 70 g Curcumin-Pulver 95 %ig und 930 g Polysorbat 80.

Außerdem kann das Polysorbat 80 ganz oder teilweise durch Polysorbat 20 ersetzt werden. So können zur Herstellung eines Curcuminsolubilisats alleine mit Polysorbat 20 beispielsweise 894 g Polysorbat 80 und 106 g 95%iges Curcumin-Pulver eingesetzt werden. Das Polysorbat 20 wird auf etwa 63 °C bis etwa 67 °C erwärmt. Unter Rühren wird das Curcuminpulver langsam zum Polysorbat 80 zugegeben. Während der Zugabe des Curcuminpulvers wird weiter auf etwa 83 °C bis etwa 87 °C erwärmt. Das entstehende Solubilisat wird langsam auf unterhalb etwa 45 °C abgekühlt und ist dann bereit zur Abfüllung.

Die Herstellung dieser Varianten entspricht ansonsten der oben beschriebenen. So können bis zu etwa 11 %ige Solubilisate hergestellt werden.

### 1,5%iges Serrapeptase-Solubilisat

Es werden
15g Serrapeptase:
Serratiopeptidase 20.000 U/mg = 300 000 000 U,
15g Wasser
16,5 g MCT Öl
953,5 g Polysorbat 80
verwendet.

Bei einer Temperatur im Bereich zwischen 18 und 22°C wird Wasser mit Serrapeptase gemischt und die Mischung homogenisiert. Das bedeutet, die Serrapeptase wird weitestgehend gleichmäßig im Wasser verteilt. So werden die Voraussetzungen dafür geschaffen, dass sich die Serrapeptase weitestgehend vollständig im Wasser lösen kann. Unter Erwärmung auf eine Temperatur im Bereich von 83 und 87°C wird MCT Öl unter ständigem Rühren in die Wasser-Serrapeptase-Mischung eingearbeitet. Dabei wird so stark gerührt, dass sich die Serrapeptase gleichmäßig in Wasser löst. Bei unveränderter Temperatur wird Polysorbat 80 unter Rühren zugegeben und homogenisiert. Dabei wird so stark gerührt, dass eine gleichmäßige Verteilung des Polysorbat 80 erfolgt. Das Produkt wird auf eine Temperatur unterhalb von 60°C abgekühlt und abgefüllt. Es wird dann bei maximal 25°C dunkel gelagert.

300.000 U/g entsprechen 15 mg/g 1,5%iger Serrepaptase in enzymatischen Units. Bei einer Verdünnung in Wasser von 1:50 wurde die Trübung dieses Solubilisats unter physiologischen Bedingungen bei pH 1,1 und 37°C bestimmt. Es ergab sich ein Wert von 1,8 FNU.

Für eine Partikelgrößenanalyse des Serrapeptase-Solubilisats wurde dieses Solubilisat dieses zunächst im Verhältnis 1:500 mit destilliertem Wasser verdünnt und unter ständigem Rühren mit einem Magnetrührer und mit Hilfe einer Heizplatte auf 37°C gebracht. Anschließend wurde der pH-Wert mit 32 %iger Salzsäure auf 1,1 eingestellt. Die Proben wurden im Anschluss sofort vermessen. Die Ergebnisse sind in der folgenden Tabelle zusammengestellt. Dabei wurden die Daten zweier Messungen gemittelt.

| | d₁₀ (nm) | d₅₀ (nm) | d₉₀ (nm) | d₉₉ (nm) |
|---|---|---|---|---|
| Intensitätsverteilung | 9,23 | 10,47 | 12,23 | 13,56 |
| Volumenverteilung | 9, 10 | 10, 18 | 11,82 | 13,12 |

### 10%iges Resveratrol-Solubilisat

Es werden
100 g Resveratrol
45 g MCT Öl
600 g Polysorbat 80
180 g Polysorbat 20 und
75 g Misch-Tocopherol
verwendet.

Bei einer Temperatur im Bereich zwischen 18 und 22°C werden die Polysorbate, das Misch-Tocopherol und das MCT Öl miteinander gemischt und homogenisiert. Dabei wird so stark gerührt, dass sich die Komponenten gleichmäßig verteilen. Unter Erwärmung auf eine Temperatur im Bereich von 83 und 87°C wird das Resveratrol zu der Mischung bzw. Lösung aus Polysorbat MCT Öl und Misch-Tocopherol gegeben. Dabei wird so stark gerührt, dass das Resveratrol gleichmäßig in die emulgatorhaltige Vorlage eingezogen wird. Sobald ein homogenes und transparentes Produkt vorliegt, wird dieses auf eine Temperatur unterhalb von 30°C abgekühlt und abgefüllt. Das Produkt ist gelblich transparent und viskos und wird dunkel bei einer Temperatur von 25°C gelagert.

Setzt man für Therapiezwecke das Verabreichen von 3.500 mg der nativen Form des Resveratrol-Rohstoffes zugrunde, so gilt folgende Berechnung für die äquivalente Solubilisatmenge:
3 Kapselfüllungen mit je 675 mg Solubilisat entsprechen einer Menge von 2.025 mg pro Tag bzw. 200 mg Wirkstoff Resveratrol und 100 mg Misch-Tocopherol. Bei einem DV-Faktor von 1:17 gilt: 200 mg x 17 = 3.400 mg Reinsubstanz Resveratrol.

Auch für dieses Solubilisat wurde die Trübung unter physiologischen Bedingungen (pH 1,1; 37°C) bei einer Verdünnung in Wasser von 1:50 bestimmt. Es ergab sich ein Wert von 16,1 FNU.

### 5%iges Coenzym Q10-Solubilisat

Es werden
57,5 g Coenzym Q10
160 g MCT Öl und
782,5 g Polysorbat 80
verwendet.

Das Polysorbat wird auf eine Temperatur im Bereich zwischen 83°C und 87°C erwärmt. Dann wird das Coenzym Q10-Pulver unter Rühren eingearbeitet. Dabei wird so stark gerührt, dass sich die Komponenten gleichmäßig verteilen. Unter Beibehaltung der Temperatur beziehungsweise nochmalige Erwärmung auf eine Temperatur im Bereich von 83 und 87°C wird das Coenzym Q10 im Polysorbat 80 gelöst. Dann wird das MCT-Öl bei gleichbleibender Temperatur eingearbeitet. Sobald ein homogenes und transparentes Produkt vorliegt, wird dieses auf eine Temperatur unterhalb von 60°C abgekühlt und abgefüllt. Das Produkt ist orange-rot, transparent und bei Raumtemperatur teilweise fest. Es wird dunkel bei einer Temperatur von 25°C gelagert.

Die Messung der Trübung unter physiologischen Bedingungen (pH 1,1; 37°C) bei einer Verdünnung in Wasser von 1:50 ergab sich ein Wert von 11,9 FNU als Mittelwert von drei Messungen (11,4 FNU; 10,5 FNU; 13,9 FNU).

Eine Partikelgrößenanalyse einer Probe von 20 mikrolitern in einer Verdünnung im Verhältnis 1:50 in einer Lösung aus Kochsalz (50 mmol/L) und Natriumazid (200 mg/L) durch die Wyatt Technology Europe GmbH im "Eclipse Program", also mittels Feldfluss-Fraktionierung, ergab einen Peak mit einem Radius von 16,5 nm für die Mizellen dieses Solubilisats. Der Durchmesser der Mizellen beträgt demnach 33 nm.

### 10 %iges α-Liponsäure-Solubilisat

Es werden

| | |
|---|---|
| 896,7 g | Polysorbat 80 |
| 103,3 g | α-Liponsäure |

verwendet. Das Polysorbat 80 wird auf 28 bis 32°C erwärmt. Unter Rühren wird das α-Liponsäure-Pulver zum Polysorbat gegeben und eingearbeitet. Die Zugabe des Pulvers erfolgt mit einer solchen Geschwindigkeit, dass es beim Rühren gleichmäßig in den Emulgator eingezogen wird. Dann erfolgt eine Erwärmung auf eine Temperatur im Bereich zwischen 83°C und 87°C. Sobald ein homogenes und transparentes Produkt vorliegt, wird dieses auf eine Temperatur unterhalb von 60°C abgekühlt und abgefüllt. Das Produkt ist gelb und viskos und wird dunkel bei einer Temperatur von 25°C gelagert.

Das Produkt kann auch mit Polysorbat 20 oder mit einer Mischung aus Polysorbat 80 und Polysorbat 20 hergestellt werden.

Bei einer Verdünnung im Verhältnis 1:50 in Wasser bei einem pH-Wert von 1,1 und einer Temperatur von 37°C hat das Solubilisat eine gemittelte Trübung von 2,9 FNU.

Eine Partikelgrößenanalyse einer Probe von 20 mikrolitern in einer Verdünnung im Verhältnis 1:50 in einer Lösung aus Kochsalz (50 mmol/L) und Natriumazid (200 mg/L) durch die Wyatt Technology Europe GmbH im "Eclipse Program", also mittels Feldfluss-Fraktionierung, ergab einen Peak mit einem Radius unterhalb von 10 nm für die Mizellen dieses Solubilisats. Der Durchmesser der Mizellen beträgt demnach maximal 20 nm.

### 10 %iges Xantho Flav-pur Solubilisat (≙ 9,2 % Xanthohumol) mit Ethanol

Es werden

| | |
|---|---|
| 100 g | Xantho Flav pur (≙ 92 g Xanthohumol), |
| 150 g | Ethanol (96 %ig) Neutralalkohol Sorte 1411U |
| und | |
| 750 g | Polysorbat 80 |

verwendet.

Zunächst wird das Xantho Flav pur-Pulver in Ethanol gelöst und dabei auf eine Temperatur im Bereich zwischen 48 und 52°C erwärmt. Es entsteht eine homogene Lösung. Polysorbat 80 wird dann unter Erwärmen auf 83 bis 87°C zur Lösung von Xantho Flav pur in Ethanol zugegeben. Die Zugabe erfolgt mit einer solchen Geschwindigkeit, dass sich die beiden Fluide durch Rühren gut homogenisieren. Das entstehende Solubilisat wird auf unter 60°C abgekühlt und abgefüllt sowie dunkel und kühl, d.h. bei Temperaturen unterhalb von 25°C gelagert.

### 15 %iges Glavonoid-Solubilisat (= 0,45 % Glabridin) mit Glycerin

Es wurden

| | |
|---|---|
| 150 g | Glavoniod (= 4,5 g Glabridin) |
| 100 g | 99%iges Glycerin |
| 750 g | Polysorbat 80 |

verwendet.

Zunächst wurden Glycerin und Glavonoid gemischt und bei einer Temperatur im Bereich von 18 bis 22°C homogenisiert. Unter Erwärmung auf 83 bis 87°C wurde Polysorbat 80 zu dem Fluid, bestehend aus Glycerin und Glavonoid, unter Rühren zugegeben. Dabei wurde so stark gerührt, dass ein homogenes Solubilisat entstand. Dieses wurde auf maximal 30°C abkühlen gelassen und abgefüllt und sodann dunkel bei einer Temperatur unterhalb von 25°C gelagert.

Die oben beschriebenen Solubilisate können verwendet werden, um das erfindungsgemäße Solubilisat mit Curcumin und zumindest einem weiteren Wirkstoff durch Mischen herzustellen. Dies wird anhand der Ausführungsbeispiele 3, 5, 6 und 7 unten beschrieben.

### Ausführungsbeispiel 1

### 5,4 % Curcumin-/6,6 % Boswelliasäure-Solubilisat

Diese Ausführungsform des erfindungsgemäßen Solubilisats wurde direkt hergestellt. Es erfolgte eine Co-Mizellierung der Wirkstoffe. Dazu wurden hier

| | |
|---|---|
| 82 g | Boswellia serrata Extrakt 80 %ig (= 65,6 g Boswelliasäure) |
| 57 g | Curcumin-Pulver 95 %ig (= 54,1 g Curcumin) |
| 70 g | Wasser |
| 350 g | Polysorbat 20 |
| 441 g | Polysorbat 80 |

verwendet.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser versetzt. Dabei wird so stark gerührt, dass das Wasser und das Ethanol gleichmäßig in der Emulgatorlösung gelöst werden. Bei unveränderter Temperatur wird das Boswellia serrata Extrakt unter Rühren in das mit Wasser verdünnte Emulgatorgemisch eingearbeitet. Die Zugabe des Boswellia serrata Extrakts erfolgt mit einer derart langsamen Geschwindigkeit, dass dieses gleichmäßig in die verdünnte Emulgatorlösung unter Rühren eingezogen wird. Anschließend wir die Temperatur unter starkem Rühren auf einen Bereich zwischen 63°C bis 67°C erhöht. Das Curcuminpulver wird unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird.

Bei einer Verdünnung im Verhältnis 1:500 in Wasser bei einem pH-Wert von 1,1 und einer Temperatur von 37°C hat das Solubilisat eine gemittelte Trübung von 1,9 FNU.

Ob eine ausreichend vollständige Homogenisierung der Komponenten zu einem erfindungsgemäßen Solubilisat abgeschlossen ist, wird bei der Herstellung aller Solubilisate im Rahmen dieser Anmeldung über Messungen der Klarheit des Produktes, welche auf die vollständige Mizellierung schließen lässt, mit Hilfe eines Laserstrahls überprüft. Eine solche Laserstrahlmessung kann beispielsweise durch Beleuchten der Probe mit Hilfe eines handelsüblichen Laserpointers, insbesondere mit einer Wellenlänge im Bereich zwischen 650 nm und 1700 nm (Spektralfarbe Rot), und anschließender Sichtkontrolle des beleuchteten beziehungsweise durchleuchteten Solubilisats. Die Kontrolle wird nicht durch Probenahme und damit außerhalb des Reaktionskessels, sondern im Reaktionskessel durchgeführt. Der Laserstrahl wird durch ein Sichtglas, welches sich auf der Vorderseite des Reaktionskessels befindet, senkrecht zum Reaktionskessel gerichtet. Wenn auf der hinteren Innenseite des Reaktionskesses vollkommen frei von Streuung nur ein Lichtpunkt erscheint, sind die entstandenen Partikelstrukturen im Reaktionskessel kleiner als die Wellenlänge des sichtbaren Lichtes und somit eine optische Bestätigung, dass der Prozess der Micellierung abgeschlossen ist.

Im Rahmen der Erfindung können die Gehalte an Curcumin und Boswelliaextrakt in den einzelnen Solubilisaten je nach Anwendungsfall auch deutlich höher als im gezeigten Beispiel eingestellt werden.

### Ausführungsbeispiel 2

### 3,3%Curcumin / 3,6%Boswelliasäure-Solubilisat mit 1,8 % Xanthohumol

Es werden

| | |
|---|---|
| 45 g | Boswellia serrata Extrakt 80 % (36 g Boswelliasäure) |
| 35 g | 95 %iges Curcumin-Pulver (33,25 g Curcumin) |
| 23 g | Xantho-Flav mit mindestens 80 % Xanthohumol (18,4 g Xanthohumol) |
| 60 g | Wasser |
| 50 g | Ethanol (96 %ig) Neutralalkohol Sorte 1411U |
| 350 g | Polysorbat 20 |
| 437 g | Polysorbat 80 |

verwendet.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser und Ethanol versetzt. Dabei wird so stark gerührt, dass das Wasser und das Ethanol gleichmäßig in der Emulgatorlösung gelöst werden. Bei unveränderter Temperatur werden das Boswellia serrata Extrakt und das Xanthohumol unter Rühren in das mit Wasser verdünnte Emulgatorgemisch eingearbeitet. Die Zugabe erfolgt mit einer derart langsamen Geschwindigkeit, dass das Boswellia serrata Extrakt und das Xanthohumol gleichmäßig in die verdünnte Emulgatorlösung unter Rühren eingezogen werden. Anschließend wir die Temperatur unter starkem Rühren auf einen Bereich zwischen 63°C bis 67°C erhöht. Das Curcuminpulver wird unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird.

Es erfolgt eine Abkühlung auf eine Temperatur von kleiner oder gleich 45°C. Das dunkelgelbe, viskose Präparat mit einem Solubilisat von Curcumin und Boswelliasäure und Xanthohumol wird dann abgefüllt und dunkel und kühl, d.h. bei unter 25°C gelagert.

Bei einer Verdünnung im Verhältnis 1:500 in Wasser bei einem pH-Wert von 1,1 und einer Temperatur von 37°C hat das Solubilisat eine gemittelte Trübung von 1,9 FNU.

Soweit nicht anders angegeben, wurden für eine Partikelgrößenanalyse eines erfindungsgemäßen Solubilisate dieses Solubilisat zunächst im Verhältnis 1:500 mit destilliertem Wasser verdünnt und unter ständigem Rühren mit einem Magnetrührer und mit Hilfe einer Heizplatte auf 37°C gebracht. Anschließend wurde der pH-Wert mit 32 %iger Salzsäure auf 1,1 eingestellt. Die Proben wurden im Anschluss sofort vermessen. Die Ergebnisse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 10,18 | 15,70 | 533,0 | 3080 |
| Volumenverteilung | 7, 90 | 10, 96 | 15,21 | 20,37 |

### Ausführungsbeispiel 3 (nicht erfindungsgemäß)

### 1,5%Curcumin- / 3%Boswelliasäure- / 2 % Xanthohumol- /0,35% Serrapeptase-Solubilisat

Es werden

| | |
|---|---|
| 250 g | 7%iges Curcuminsolubilisat |
| 250 g | 12%iges Bowellia-Solubilisat |
| 250 g | 9,2%iges Xanthohumol-Solubilisat und |
| 250 g | 1,5%iges Serrapeptase-Solubilisat |

jeweils nach den oben beschriebenen Rezepturen verwendet. Alle vier Solubilisate können zum Senken der Viskosität und damit zur besseren Fließfähigkeit auf eine Temperatur im Bereich von 50°C bis 60°C erwärmt werden. Dann werden sie durch Rühren miteinander gemischt. Sobald ein homogenes Gesamtprodukt vorliegt wird dieses gegebenenfalls auf eine Temperatur unter 60°C abgekühlt und abgefüllt.

Vor der Weiterverarbeitung wie etwa die Abfüllung in Kapseln ist es zweckmäßig, das Produkt erneut aufzurühren, um es zu homogenisieren, und dazu gegebenenfalls etwas, also auf eine Temperatur von etwa 40°C bis 50°C zu erwärmen.

Bei einer Verdünnung im Verhältnis 1:500 in Wasser bei einem pH-Wert von 1,1 und einer Temperatur von 37°C hat das Solubilisat eine gemittelte Trübung von 1,0 FNU.

Die Ergebnisse der Partikelgrößenanalyse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 9, 08 | 15, 64 | 292,7 | 615 |
| Volumenverteilung | 6, 35 | 9,36 | 14, 10 | 20, 16 |

### Ausführungsbeispiel 4

5% Glavonoid (= 1,8 % Glabridin)/
3 % Curcumin/3,5 % Xanthohumol-Solubilisat

Es werden

| | |
|---|---|
| 32 g | Curcumin-Pulver 95 % (= 30,4 g Curcumin) |
| 44 g | Xantho Flav pur-Pulver (= 35,2 g Xanthohumol) |
| 60 g | Kaneka Glavonoid (= 1,8 g Glabridin) |
| 60 g | Ethanol 96 %ig Neutralalkohol Sorte 1411U |
| 44 g | Glycerin 99,5 % |
| 760 g | Polysorbat 80 |

verwendet.

Polysorbat 80 und Glycerin werden unter Rühren gemischt und dabei auf eine Temperatur im Bereich von 48 bis 52°C erwärmt, um die Mischung gut zu homogenisieren. Ethanol wird unter derart starkem Rühren in das Polysorbat-Glycerin-Gemisch eingearbeitet, dass sich eine homogene Lösung bildet. Die Temperatur wird dabei konstant gehalten. Dann wird Xanthohumol in die Lösung aus Polysorbat, Glycerin und Ethanol eingearbeitet. Dabei wird die Temperatur auf einen Wert im Bereich zwischen 63 und 67°C erhöht. Es wird derart stark gerührt, dass sich Xanthohumol homogen mit der vorgelegten Lösung verbindet.

Anschließend wird Curcumin-Pulver in das Xanthohumol-Solubilisat eingearbeitet. Dabei wird die Temperatur auf einen Wert im Bereich zwischen 78 und 82°C erhöht. Wie bei Xanthohumol und auch beim unten beschriebenen Einarbeiten von Glavonoid wird dabei jeweils derart stark gerührt, dass sich die neu zugegebene Komponente des Solubilisats in dem vorgelegten Fluid homogen zum solubilisierten Produkt verbindet. Für die Zugabe von Glavonoid wird die Temperatur weiter auf einen Wert im Bereich zwischen 85 und 98°C erhöht.

Das Produkt ist ein Solubilisat mit co-mizelliertem Curcumin, Xanthohumol und Glavonoid. Es wird auf einen Wert von maximal 45°C unter Rühren abkühlen gelassen und dann abgefüllt.

### Ausführungsbeispiel 5 (nicht erfindungsgemäß)

### 1,5%Curcumin- / 2 % Xanthohumol- /3,5% Glavonoid (0,1 % Glabridin) / 1,2% Coenzym Q10-Solubilisat

Es werden

| | |
|---|---|
| 250 g | 7%iges Curcuminsolubilisat |
| 250 g | 9,2%iges Xanthohumol-Solubilisat |
| 250 g | 15%iges Glavonoid-Solubilisat und |
| 250 g | 5%iges Coenzym Q10-Solubilisat |

jeweils nach den oben beschriebenen Rezepturen verwendet.

Alle vier Solubilisate können zum Senken der Viskosität und damit zur besseren Fließfähigkeit auf eine Temperatur im Bereich von 50°C bis 60°C erwärmt werden. Dann werden sie durch Rühren miteinander gemischt. Sobald ein homogenes Gesamtprodukt vorliegt wird dieses gegebenenfalls auf eine Temperatur unter 60°C abgekühlt und abgefüllt.

Vor der Weiterverarbeitung wie etwa die Abfüllung in Kapseln ist es zweckmäßig, das Produkt erneut aufzurühren, um es zu homogenisieren, und dazu gegebenenfalls etwas, also auf eine Temperatur von etwa 40°C bis 50°C zu erwärmen.

Die Ergebnisse der Partikelgrößenanalyse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 8,22 | 10,88 | 14,80 | 450 |
| Volumenverteilung | 7,78 | 10, 06 | 13,10 | 16, 39 |

### Ausführungsbeispiel 6 (nicht erfindungsgemäß)

### 1,3%Curcumin- / 1,6 % Xanthohumol- /3% Glavonoid (0,09 % Glabridin) / 1% Coenzym Q10- / 2% α-Liponsäure-Solubilisat

Es werden

| | |
|---|---|
| 200 g | 7%iges Curcuminsolubilisat |
| 200 g | 9,2%iges Xanthohumol-Solubilisat |
| 200 g | 15%iges Glavonoid-Solubilisat |
| 200 g | 5%iges Coenzym Q10-Solubilisat und |
| 200 g | 10%iges a-Liponsäure-Solubilisat |

jeweils nach den oben beschriebenen Rezepturen verwendet.

Alle fünf Solubilisate können zum Senken der Viskosität und damit zur besseren Fließfähigkeit auf eine Temperatur im Bereich von 50°C bis 60°C erwärmt werden. Dann werden sie durch Rühren miteinander gemischt. Sobald ein homogenes Gesamtprodukt vorliegt wird dieses gegebenenfalls auf eine Temperatur unter 60°C abgekühlt und abgefüllt.

Vor der Weiterverarbeitung wie etwa die Abfüllung in Kapseln ist es zweckmäßig, das Produkt erneut aufzurühren, um es zu homogenisieren, und dazu gegebenenfalls etwas, also auf eine Temperatur von etwa 40°C bis 50°C zu erwärmen.

Die Ergebnisse der Partikelgrößenanalyse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 8, 85 | 11, 64 | 17,05 | 981 |
| Volumenverteilung | 7,16 | 9,41 | 12,36 | 15, 43 |

### Ausführungsbeispiel 7 (nicht erfindungsgemäß)

### 0,8öCurcumin- 1 1,5% Boswelliasäuren- /1 % Xanthohumol- / 1,8% Glavonoid- (0,05 % Glabridin) / 0,18% Serrapeptase- / 1,2% Resveratrol- / 0,6% Coenzym Q10- / 1,2% α-Liponsäure-Solubilisat

Es werden

| | |
|---|---|
| 125 g | 7%iges Curcuminsolubilisat |
| 125 g | 12%iges Boswellia-Solubilisat |
| 125 g | 9,2%iges Xanthohumol-Solubilisat |
| 125 g | 15%iges Glavonoid-Solubilisat |
| 125 g | 1,5%iges Serrapeptase-Solubilisat |
| 125 g | 10%iges Resveratrol-Solubilisat |
| 125 g | 5%iges Coenzym Q10-Solubilisat und |
| 125 g | 10%iges a-Liponsäure-Solubilisat |

jeweils nach den oben beschriebenen Rezepturen verwendet. Alle acht Solubilisate können zum Senken der Viskosität und damit zur besseren Fließfähigkeit auf eine Temperatur im Bereich von 50°C bis 60°C erwärmt werden. Dann werden sie durch Rühren miteinander gemischt. Sobald ein homogenes Gesamtprodukt vorliegt wird dieses gegebenenfalls auf eine Temperatur unter 60°C abgekühlt und abgefüllt.

Vor der Weiterverarbeitung wie etwa die Abfüllung in Kapseln ist es zweckmäßig, das Produkt erneut aufzurühren, um es zu homogenisieren, und dazu gegebenenfalls etwas, also auf eine Temperatur von etwa 40°C bis 50°C zu erwärmen.

Bei einer Verdünnung im Verhältnis 1:50 in Wasser bei einem pH-Wert von 1,1 und einer Temperatur von 37°C hat das Solubilisat eine gemittelte Trübung von 12,5 FNU.

Die Ergebnisse der Partikelgrößenanalyse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 8, 09 | 10,91 | 15,19 | 425 |
| Volumenverteilung | 6, 90 | 9,26 | 12,37 | 15,71 |

### Ausführungsbeispiel 8 (nicht erfindungsgemäß)

### 0,8öCurcumin- 1 1,5% Boswelliasäuren- /1 % Xanthohumol- / 1,8% Glavonoid- (0,05 % Glabridin) / 0,18% Serrapeptase- / 1,2% Resveratrol- / 0,6% Coenzym Q10- / 1,2% α-Liponsäure-Solubilisat in Direktherstellung

Es werden

| | |
|---|---|
| 9,375 g | Curcuminpulver 95%ig |
| 19 g | Boswellia Serrata Extrakt (15,2 Boswelliasäure) |
| 12,5 g | Xantho Flav-Pulver (mindestens 80 % Xanthohumol = 10 g Xanthohumol) |

| | |
|---|---|
| 18,75 | Glavonoid (0,56 g Glabridin) |
| 1,875 g | Serrepeptase (37 500 000 U) |
| 12,5 g | Resveratrol |
| 7,19 g | Co-Enzym Q10 |
| 12,5 g | α-Liponsäure |
| 7,875 g | Wasser |
| 18,75 g | Ethanol |
| 12,5 g | Glycerin |
| 9,375 g | Mischtocopherol |
| 27,685 g | MCT-Öl |
| 122,5 g | Polysorbat 20 und |
| 707,225 g | Polysorbat 80 |

verwendet.

Polysorbat 80 wird mit α-Liponsäure bei einer Temperatur im Bereich zwischen 18 und 22°C gemischt. Dabei wird so stark gerührt, dass eine homogene Mischung entsteht. Separat dazu wird die Serrapeptase bei einer Temperatur im Bereich von 18 bis 22°C in gleicher Weise in Wasser gelöst. Zu der Mischung bzw. Lösung aus Serrepeptase und Wasser werden langsam und nacheinander unter ständigem Rühren die weiteren Zutaten Ethanol, Xanthohumol, Curcumin, Boswellia, Glavonoid, Resveratrol, Co-Enzym Q10, Misch-Tocopherol, Glycerin, MCT-Öl und Polysorbat 20 zudosiert. Die Temperatur liegt dabei weiter im Bereich zwischen 18 und 22°C. Es wird auf eine gute Durchmischung und Homogenität des Produktes geachtet. Gegebenenfalls werden Pausen zwischen der Zugabe einer Substanz und der Zugabe der nächsten Substanz eingelegt. Dabei wird derart langsam zudosiert und gerührt, dass die jeweils zuzugebende Zutat gleichmäßig in die Vorlage eingearbeitet wird.

Als nächstes werden beide Mischungen, das heißt die Polysorbat 80 und α-Liponsäure-Vorlage sowie die restliche Mischung aller anderen Zutaten zusammengegeben und bei einer Temperatur im Bereich von 18 bis 22°C weiter gerührt. Es entsteht dabei eine homogene pastöse Masse ähnlich einem "Brei". Dieser wird auf eine Temperatur im Bereich von 85°C bis 89°C erwärmt. Die Erwärmung wird unter ständigem Rühren derart langsam durchgeführt, dass der sich erwärmende

"Brei" stets möglichst homogen durchmischt bleibt. Nach Abkühlen auf eine Temperatur unterhalb von 60°C wird das Produkt abgefüllt. Es ist dunkel und viskos und wird dunkel bei Temperaturen von maximal 25°C gelagert. Vor der Weiterverarbeitung, wie etwa die Abfüllung in Kapseln, ist es zweckmäßig, das Produkt erneut aufzurühren, um es zu homogenisieren und dazu ggf. etwas, also auf eine Temperatur von etwa 40 bis 50°C, zu erwärmen.

Bei einer Verdünnung im Verhältnis 1:50 in Wasser bei einem pH-Wert von 1,1 und einer Temperatur von 37°C hat das Solubilisat eine Trübung von 1,0 FNU.

Die Ergebnisse der Partikelgrößenanalyse sind in der folgenden Tabelle zusammengestellt.

| | d₁₀ (nm) | d₅₀ (nm) | d₉₀ (nm) | d₉₉ (nm) |
|---|---|---|---|---|
| Intensitätsverteilung | 8, 66 | 11,01 | 14,58 | 220,4 |
| Volumenverteilung | 7, 96 | 9, 87 | 12,59 | 15, 52 |

### Ausführungsbeispiel 9

### 3öCurcumin- / 3,2% Boswelliasäuren- /1,6% Xanthohumol- / 1% CBD-Öl-Solubilisat

Es werden

| | |
|---|---|
| 40,5 g | Boswellia serrata extrakt 80%ig (32,4% Boswelliasäure) |
| 31,5 g | Curcuminpulver 95%ig (29,925 g Curcumin) |
| 20,7 g | Xantho Flav Pulver mit mindestens 80% Xanthohumol (16,5 g Xanthohumol) |
| 54 g | Wasser |
| 45 g | Ethanol |
| 315 g | Polysorbat 20 |
| 483 g | Polysorbat 80 |
| 10 g | CBD-Öl: Cannabidiol, 30%ig, |

verwendet. Cannabidiol (CBD) ist ein kaum psychoaktives Cannabinoid aus dem weiblichen Hanf *Cannabis sativa* beziehungsweise *Cannabis indica.* Eingesetzt wurde ein nicht THC-freies CBD-Öl Das bedeutet, Spuren von THC können darin enthalten sein. Tetrahydrocannabinol (THC) ist für die berauschenden Effekte von Hanfpflanzen verantwortlich.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser und Ethanol versetzt. Dabei wird so stark gerührt, dass das Wasser und das Ethanol gleichmäßig in der Emulgatorlösung gelöst werden. Bei unveränderter Temperatur werden das Boswellia serrata Extrakt und das Xanthohumol unter Rühren in das mit Wasser verdünnte Emulgatorgemisch eingearbeitet. Die Zugabe erfolgt mit einer derart langsamen Geschwindigkeit, dass das Boswellia serrata Extrakt und das Xanthohumol gleichmäßig in die verdünnte Emulgatorlösung unter Rühren eingezogen werden. Anschließend wir die Temperatur unter starkem Rühren auf einen Bereich zwischen 63°C bis 67°C erhöht. Das Curcuminpulver wird unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird. Anschließend wird das CBD-Öl in die Mischung eingeareitet. Dabei wird so stark gerührt, dass das CBD-Öl gleichmäßig in der Vorlage verteilt und homogenisiert wird.

Es erfolgt eine Abkühlung auf eine Temperatur von kleiner oder gleich 45°C. Das dunkelgelbe, viskose Präparat mit einem Solubilisat von Curcumin und Boswelliasäure und Xanthohumol und CBD-Öl wird dann abgefüllt und dunkel und kühl, d.h. bei unter 25°C gelagert. Das Solubilisat sowie die wässrige Lösung daraus sind stabil homogen beziehungsweise genauso kristallklar löslich wie das Solubilisat gemäß Ausführungsbeispiel 2.

Es ist dem Fachmann ersichtlich, dass die Erfindung nicht auf die vorstehend beschriebenen Beispiele beschränkt ist, sondern vielmehr in vielfältiger Weise variiert werden kann. Insbesondere können die Merkmale der einzeln dargestellten Beispiele auch miteinander kombiniert oder gegeneinander ausgetauscht werden.

## Patentansprüche

1. Solubilisat enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt,
zur Verwendung bei der Vorbeugung vor mit einer Entzündung einhergehenden Krankheiten, Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, zum Senken von Cholesterin, insbesondere LDL-Cholesterin, und/oder von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel zur Behandlung von und/oder Vorbeugung vor Fettleber, Friedreich-Ataxie, lysomale Krankheiten, insbesondere Morbus Tay-Sachs, Arteriosklerose, Herzkrankheiten, Arthritis.

2. Solubilisat nach Anspruch 1
zur Anwendung als entzündungshemmendes Nahrungsergänzungsmittel und/oder als Arzneimittel mit einer Wirkung gegen Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, als cholesterinsekendes Arzneimittel, insbesondere betreffend LDL-Cholesterin, und/oder als Arzneimittel mit einer Wirkung zur Senkung von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel mit einer Wirkung gegen Fettleber, Friedreich-Ataxie, lysomale Krankheiten, insbesondere Morbus Tay-Sachs, Arteriosklerose, Herzkrankheiten, Arthritis.

3. Solubilisat enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt,
zur Anwendung im Bereich der Landwirtschaft, Fischzucht und/oder im Gartenbau und/oder im Bereich der Lebensmittelhygiene und/oder zur Anwendung als Desinfektionsmittel und/oder im Bereich der Verpackung, bevorzugt bei der Verpackung von Rindfleisch, Geflügel oder Fisch, und/oder zur Anwendung als Desinfektionsmittel.

4. Solubilisat zur Verwendung nach Anspruch 1 oder 2 oder Solubilisat nach Anspruch 3,
**dadurch gekennzeichnet, dass**
das Solubilisat bis zu 20 Gew.-%, bevorzugt bis zu 15 Gew.-% Ethanol enthält
und/oder dass
das Solubilisat bis zu 25 Gew.-%, bevorzugt zwischen 12 Gew.-% und 20 Gew.-%, besonders bevorzugt bis zu 10 Gew.-% Glycerin enthält
und/oder dass
das Solubilisat zusätzlich bis zu 10 Gew.-%, bevorzugt bis zu 7 Gew.-% Wasser enthält
und/oder dass
die Durchmesserverteilung der Micellen in einer Verdünnung des Solubilisats mit destilliertem Wasser im Verhältnis 1:500 bei physiologischen Bedingungen (pH 1,1 und 37°C) von etwa d₁₀=6 nm bis etwa d₉₀=20 nm reicht
und/oder dass
die Trübung des Solubilisats kleiner als 25 FNU, bevorzugt kleiner als 3 FNU ist gemessen durch Streulichtmessung mit Infrarotlicht nach den Vorschriften der Norm ISO 7027 bei einer Verdünnung des Solubilisats im Verhältnis 1:50 oder 1:500 in Wasser bei physiologischen Bedingungen (pH 1,1 und 37°C).

5. Kapsel gefüllt mit einem Solubilisat zur Verwendung nach Anspruch 1 oder 2 oder 4 oder mit einem Solubilisat nach Anspruch 3 oder 4,
**dadurch gekennzeichnet, dass**
die Kapsel als Weichgelatinekapsel oder Hartgelatinekapsel oder als weiche, gelatinefreie Kapsel oder als harte, gelatinefreie Kapsel, zum Beispiel als Cellulose-Kapsel, ausgebildet ist.

6. Fluid enthaltend ein Solubilisat zur Verwendung nach Anspruch 1 oder 2 oder 4 oder ein Solubilisat nach Anspruch 3 oder 4,
**dadurch gekennzeichnet, dass**
das Fluid aus der Gruppe ausgewählt ist, welche Lebensmittel, Nahrungsergänzungsmittel, Getränke, Kosmetika und pharmazeutische Produkte umfasst
und/oder dass
das Fluid eine wässrige Verdünnung des Solubilisats umfasst.

7. Solubilisat nach einem der Ansprüche 1 bis 4 oder Kapsel nach Anspruch 5 oder Fluid nach Anspruch 6 zur oralen Anwendung in einem Verfahren zur Vorbeugung vor mit einer Entzündung einhergehenden Krankheiten, Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, zum Senken von Cholesterin, insbesondere LDL-Cholesterin, und/oder von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel zur Behandlung von und/oder Vorbeugung vor Fettleber, Friedreich-Ataxie, lysomale Krankheiten, insbesondere Morbus Tay-Sachs, Arteriosklerose, Herzkrankheiten, Arthritis.

8. Solubilisat zur Anwendung gemäß Anspruch 7,
**dadurch gekennzeichnet, dass**
das Solubilisat dem Nahrungsergänzungsmittel-Konsumenten oder Patienten in einer Curcumin-Dosis im Bereich von 0,5 mg/kg Körpergewicht bis 1 mg/ kg Körpergewicht, bevorzugt mit einer Dosis von 0,81 mg/kg Körpergewicht, insbesondere einmal täglich, verabreicht wird
und/oder dass.
das Solubilisat dem Nahrungsergänzungsmittel-Konsumenten oder Patienten in einer Boswellia-Dosis im Bereich von 1 mg/kg Körpergewicht bis 2 mg/kg Körpergewicht, bevorzugt mit einer Dosis von 1,62 mg/kg Körpergewicht, insbesondere einmal täglich, verabreicht wird
und/oder dass
das Solubilisat dem Nahrungsergänzungsmittel-Konsumenten oder Patienten in einer Xanthohumol-Dosis im Bereich von 0,5 mg/kg Körpergewicht bis 1 mg/kg Körpergewicht, bevorzugt mit einer Dosis von 0,81 mg/kg Körpergewicht, verabreicht wird.

9. Verfahren zum Herstellen eines Solubilisats enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt, insbesondere eines Solubilisats nach Anspruch 4, mit folgenden Schritten
a) Vorlegen von Polysorbat 80 und/oder Polysorbat 20 und/oder eine Mischung aus Polysorbat 20 und Polysorbat 80
b) Zugabe zumindest eines weiteren Wirkstoffes, insbesondere *Boswellia* serrata-Extrakt und/oder Xanthohumol,
c) Zugabe von Curcumin Pulver
wobei
in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei
in Schritt b) die Temperatur unverändert bleibt gegenüber Schritt a) oder eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt,
und wobei
in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

10. Verfahren nach Anspruch 9,
wobei vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C,
durchgeführt wird.

11. Verfahren nach Anspruch 9 oder 10,
wobei in Schritt
b1) auch die Zugabe von Ethanol bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C,
durchgeführt wird.

12. Verfahren zum Herstellen eines Solubilisats enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt, insbesondere eines Solubilisats nach Anspruch 4
mit folgenden Schritten
a) Herstellen eines ersten Ansatzes durch Vorlegen von Polysorbat 80 und/oder Polysorbat 20 und/oder eine Mischung aus Polysorbat 20 und Polysorbat 80 und von mindestens einem ersten Wirkstoff, insbesondere α-Liponsäure,
b) Herstellen eines zweiten Ansatzes durch Vorlegen von Wasser und zumindest einem weiteren Wirkstoff, insbesondere Serrapeptase
c) Zugabe von zumindest einem weiteren Wirkstoff, insbesondere Xanthohumol und/oder, Boswellia serrata-Extrakt und/oder Glavonoid und/oder Resveratrol und/oder Coenzym Q10, und Zugabe von Curcuminpulver zu dem zweiten Ansatz aus Schritt b)
d) Vereinigen des ersten Ansatzes aus Schritt a) und des zweiten Ansatzes aus Schritt c),
wobei die Temperatur während der Durchführung der Schritte a) bis d) im Bereich von 18°C bis 22°C liegt,
e) Erwärmung auf eine Temperatur im Bereich von 80°C bis 97°C, bevorzugt auf eine Temperatur im Bereich von 83°C bis 92°C, besonders bevorzugt auf eine Temperatur im Bereich von 85°C und 89°C.

13. Verfahren nach Anspruch 12,
wobei in Schritt
c) auch die Zugabe von Ethanol und/oder Glycerin und/oder MCT-Öl und/oder Polysorbat 20 und/oder Polysorbat 80 und/oder einer Mischung von Polysorbat 20 und Polysorbat 80 erfolgt.

14. Verfahren zum Herstellen eines Solubilisats enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt, insbesondere eines Solubilisats nach Anspruch 4
mit folgenden Schritten
a) Vorlegen von Polysorbat 80 und/oder Polysorbat 20 und/oder eine Mischung aus Polysorbat 20 und Polysorbat 80 und von Glycerin und von Ethanol,
b) Zugabe zumindest eines weiteren Wirkstoffes, insbesondere eines ethanolischen Auszugs der Hartharze aus Hopfen,
wobei
in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei
in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt,
c) Zugabe von Curcuminpulver bei einer Temperatur im Bereich von 70°C bis 92°C, bevorzugt bei einer Temperatur im Bereich von 75°C bis 87°C, besonders bevorzugt bei einer Temperatur im Bereich von 78°C und 82°C,
d) Zugabe von Glavonoid unter Erwärmung auf eine Temperatur im Bereich von 80°C bis 97°C, bevorzugt auf eine Temperatur im Bereich von 83°C bis 92°C, besonders bevorzugt auf eine Temperatur im Bereich von 85°C und 89°C.

15. Verfahren zum Herstellen eines Solubilisats enthaltend, insbesondere bestehend aus,
Curcumin mit einem Anteil von 3 Gew.-% bis 7 Gew.-%,
und zumindest einen weiteren Wirkstoff,
welcher eine Substanz oder mehrere Substanzen umfasst, welche aus der Gruppe ausgewählt ist oder sind, welche Xanthohumol, Pflanzenextrakte, insbesondere aus dem Harz des Weihrauchbaumes, aus Süßholz, Cannabinoide, Enzyme, insbesondere Serrapeptase, Coenzym Q10, α-Liponsäure, Resveratrol, umfasst,
sowie
zumindest einen Emulgator mit einem HLB-Wert im Bereich kleiner 18, bevorzugt zwischen 13 und 18, nämlich Polysorbat 80 oder Polysorbat 20 oder einer Mischung aus Polysorbat 20 und Polysorbat 80,
wobei der Polysorbatanteil bei mindestens 70 Gew-%, bevorzugt im Bereich zwischen 75 Gew-% und 95 Gew-%, besonders bevorzugt im Bereich zwischen 79 Gew-% und 88 Gew-% liegt, insbesondere eines Solubilisats nach Anspruch 4,
durch Mischen eines Curcuminsolubilisats und zumindest eines weiteren Wirkstoff-Solubilisats, insbesondere im Mengenverhältnis 1:1 der einzelnen Solubilisate zueinander.

## Claims

1. A solubilizate, containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%;
for use in the treatment and/or prevention of diseases involving inflammation, cancer, Alzheimer's, Parkinson's, obesity, high cholesterol, elevated blood sugar, diabetes, metabolic syndrome, and/or autoimmune diseases, multiple sclerosis (MS), for reducing visceral fat, for thermogenesis, for lowering cholesterol, in particular LDL cholesterol, and/or glucose in the blood and/or triglycerides in the blood, for improving macular pigment density, for reducing oxidative stress and/or for reducing the accumulation of fat in the hepatocytes, in particular as a pharmaceutical drug for treating and/or preventing fatty liver disease, Friedreich's ataxia, lysosomal diseases, in particular Tay-Sachs disease, arteriosclerosis, heart diseases, arthritis.

2. The solubilizate of claim 1,
for use as an anti-inflammatory dietary supplement and/or as a pharmaceutical drug with an effect against cancer, Alzheimer's, Parkinson's, obesity, high cholesterol, elevated blood sugar, diabetes, metabolic syndrome, and/or autoimmune diseases, multiple sclerosis (MS), for lowering visceral fat, for thermogenesis, as a cholesterol-lowering pharmaceutical drug, in particular with respect to LDL cholesterol, and/or as a pharmaceutical drug with an effect for lowering glucose in the blood and/or triglycerides in the blood, for improving macular pigment density, for reducing oxidative stress and/or for reducing the accumulation of fat in the hepatocytes, in particular as a pharmaceutical drug with an effect against fatty liver disease, Friedreich's ataxia, lysosomal diseases, in particular Tay-Sachs disease, arteriosclerosis, heart diseases, arthritis.

3. A solubilizate, containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%,
for use in agriculture, fish farming and/or horticulture and/or in the field of food hygiene and/or for use as a disinfectant and/or in the field of packaging, preferably in the packaging of beef, poultry or fish, and/or for use as a disinfectant.

4. The solubilizate for use according to claim 1 or 2 or solubilizate according to claim 3, **characterized in that**
the solubilizate contains up to 20 wt.%, preferably up to 15 wt.% of ethanol
and/or **in that**
the solubilizate contains up to 25 wt.%, preferably between 12 wt.% and 20 wt.%, most preferably up to 10 wt.% of glycerol
and/or **in that**
the solubilizate additionally contains up to 10 wt.%, preferably up to 7 wt.% of water
and/or **in that**
the diameter distribution of the micelles in a dilution of the solubilizate with distilled water in a ratio of 1:500 under physiological conditions (pH 1.1 and 37 °C) is in a range from about d₁₀ = 6 nm to about d₉₀ = 20 nm
and/or **in that**
turbidity of the solubilizate is less than 25 FNU, preferably less than 3 FNU, measured by scattered light measurement using infrared light according to the specifications of the ISO 7027 standard at a dilution of the solubilizate in a ratio of 1:50 or 1:500 in water under physiological conditions (pH 1.1 and 37 °C).

5. A capsule filled with a solubilizate for use according to claim 1 or 2 or 4 or with a solubilisate according to claim 3 or 4,
**characterized in that**
the capsule is in the form of a soft gelatin capsule or a hard gelatin capsule or a soft gelatin-free capsule or a hard gelatin-free capsule, for example a cellulose capsule.

6. A fluid, containing a solubilizate for use according to claim 1 or 2 or 4 or with a solubilisate according to claim 3 or 4,
**characterized in that**
the fluid is selected from the group consisting of foods, dietary supplements, beverages, cosmetics, and pharmaceutical products
and/or **in that**
the fluid comprises an aqueous dilution of the solubilizate.

7. A solubilizate according to one of claims 1 to 4 or capsule according to claim 5 or fluid according to claim 6 for oral use in a method for treating and/or preventing diseases involving inflammation, cancer, Alzheimer's, Parkinson's, obesity, high cholesterol, elevated blood sugar, diabetes, metabolic syndrome, and/or autoimmune diseases, multiple sclerosis (MS), for reducing visceral fat, for thermogenesis, for lowering cholesterol, in particular LDL cholesterol, and/or glucose in the blood and/or triglycerides in the blood, for improving macular pigment density, for reducing oxidative stress and/or for reducing the accumulation of fat in the hepatocytes, in particular as a pharmaceutical drug for treating and/or preventing fatty liver disease, Friedreich's ataxia, lysosomal diseases, in particular Tay-Sachs disease, arteriosclerosis, heart diseases, arthritis.

8. A solubilizate for use according to claim 7,
**characterized in that**
the solubilizate is administered to the dietary supplement consumer or patient in a dose of curcumin ranging from 0.5 mg/kg body weight to 1 mg/kg body weight, preferably in a dose of 0.81 mg/kg body weight, in particular once daily and/or **in that**
the solubilizate is administered to the dietary supplement consumer or patient in a Boswellia dose ranging from 1 mg/kg body weight to 2 mg/kg body weight, preferably in a dose of 1.62 mg/kg body weight, in particular once daily
and/or **in that**
the solubilizate is administered to the dietary supplement consumer or patient in a xanthohumol dose ranging from 0.5 mg/kg body weight to 1 mg/kg body weight, preferably in a dose of 0.81 mg/kg body weight.

9. A method for producing a solubilizate containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%, in particular a solubilizate according to claim 4;
comprising the following steps
(a) providing polysorbate 80 and/or polysorbate 20 and/or a mixture of polysorbate 20 and polysorbate 80;
(b) adding at least one further active substance, in particular *Boswellia serrata* extract and/or xanthohumol;
(c) adding curcumin powder;
wherein step (a) comprises heating to a temperature in a range from 40 °C to 62 °C, preferably to a temperature in a range from 45 °C to 57 °C, most preferably to a temperature in a range from 48 °C and 52 °C; and
wherein step (b) comprises keeping the temperature unchanged compared to step a), or heating to a temperature in a range from 60 °C to 75 °C, preferably to a temperature in a range from 61 °C to 70 °C, most preferably to a temperature in a range from 63 °C and 67 °C; and
wherein step (c) comprises heating to a temperature in a range from 82 °C to 97 °C, preferably to a temperature in a range from 83 °C to 92 °C, most preferably to a temperature in a range from 85 °C and 89 °C.

10. The method of claim 9, wherein prior to step (b), a step
(b1) is performed, comprising adding water at a temperature in a range from 40 °C to 62 °C, preferably at a temperature in a range from 45 °C to 57 °C, most preferably at a temperature in a range from 48 °C to 52 °C.

11. The method of claim 9 or 10, wherein step
(b1) further comprises adding ethanol at a temperature in a range from 40 °C to 62 °C, preferably at a temperature in a range from 45 °C to 57 °C, most preferably at a temperature in a range from 48 °C to 52 °C.

12. A method for producing a solubilizate containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%, in particular solubilizate according to claim 4;
with the following steps
(a) preparing a first preparation by providing polysorbate 80 and/or polysorbate 20 and/or a mixture of polysorbate 20 and polysorbate 80 and at least a first active substance, in particular α-lipoic acid;
(b) preparing a second preparation by providing water and at least one further active substance, in particular serrapeptase;
(c) adding at least one further active substance, in particular xanthohumol and/or *Boswellia serrata* extract and/or glavonoid and/or resveratrol and/or coenzyme Q₁₀, and adding curcumin powder to the second preparation from step (b);
(d) combining the first preparation from step (a) and the second preparation from step c); wherein the temperature is in a range from 18 °C to 22 °C during the execution of steps (a) through (d);
(e) heating to a temperature in a range from 80 °C to 97 °C, preferably to a temperature in a range from 83 °C to 92 °C, most preferably to a temperature in a range from 85 °C to 89 °C.

13. The method of claim 12, wherein step
(c) further comprises adding ethanol and/or glycerol and/or MCT oil and/or polysorbate 20 and/or polysorbate 80 and/or a mixture of polysorbate 20 and polysorbate 80.

14. A method for producing a solubilizate containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%, in particular solubilizate according to claim 4,
with the following steps
(a) providing polysorbate 80 and/or polysorbate 20 and/or a mixture of polysorbate 20 and polysorbate 80 and of glycerol and of ethanol;
(b) adding at least one further active substance, in particular an ethanolic extract of hard resins from hops;
wherein
step (a) comprises heating to a temperature in a range from 40 °C to 62 °C, preferably to a temperature in a range from 45 °C to 57 °C, most preferably to a temperature in a range from 48 °C to 52 °C;
and wherein
step (b) comprises heating to a temperature in a range from 60 °C to 75 °C, preferably to a temperature in a range from 61 °C to 70 °C, most preferably to a temperature in a range from 63 °C to 67 °C; and
(c) adding curcumin powder at a temperature in a range from 70 °C to 92 °C, preferably at a temperature in a range from 75 °C to 87 °C, most preferably at a temperature in a range from 78 °C to 82 °C;
(d) adding glavonoid under heating to a temperature in a range from 80 °C to 97 °C, preferably to a temperature in a range from 83 °C to 92 °C, most preferably to a temperature in a range from 85 °C to 89 °C.

15. A method for producing a solubilizate containing and in particular consisting of
curcumin in a content of 3 wt.% to 7 wt.%;
and at least one further active substance,
which comprises one or more substances selected from the group consisting of xanthohumol, plant extracts, in particular from the resin of the frankincense tree, licorice, cannabinoids, enzymes, in particular serrapeptase, coenzyme Q₁₀, α-lipoic acid, resveratrol; and
at least one emulsifier having an HLB value in a range below 18, preferably between 13 and 18, namely polysorbate 80 or polysorbate 20 or a mixture of polysorbate 20 and polysorbate 80;
wherein the polysorbate content is at least 70 wt.%, preferably in the range between 75 wt.% and 95 wt.%, most preferably in the range between 79 wt.% and 88 wt.%, in particular a solubilizate according to claim 4,
by mixing a curcumin solubilizate and a solubilizate of at least one further active substance, in particular in a quantitative ratio of 1:1 of the individual solubilizates.

## Revendications

1. Solubilisat contenant, en particulier composé
de curcumine dans une proportion de 3 % à 7 % en poids,
et d'au moins un autre principe actif,
qui comprend une ou plusieurs substances qui est ou sont choisies dans le groupe comprenant le xanthohumol, des extraits de plantes, notamment de résine d'arbre à encens, de réglisse, les cannabinoïdes, les enzymes, en particulier la serrapeptase, la coenzyme 010, l'acide alpha-lipoïque, le resvératrol,
ainsi que
d'au moins un émulsifiant ayant une valeur HLB inférieure à 18, préférablement comprise entre 13 et 18,
à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement comprise entre 79 % et 88 % en poids,
destiné à une utilisation dans la prévention des maladies inflammatoires, du cancer, de la maladie d'Alzheimer, la maladie de Parkinson, l'obésité, l'hypercholestérolémie, l'hyperglycémie, du diabète, du syndrome métabolique et/ou des maladies auto-immunes, de la sclérose en plaques (SEP), pour réduire la graisse viscérale, pour la thermogenèse, pour réduire le cholestérol, en particulier le cholestérol LDL, et/ou le glucose dans le sang et/ou les triglycérides dans le sang, pour améliorer la densité du pigment maculaire, pour diminuer le stress oxydatif et/ou pour diminuer l'accumulation de graisse dans les hépatocytes, en particulier comme médicament destiné au traitement et/ou à la prévention de la stéatose hépatique, l'ataxie de Friedreich, des maladies lysomales, en particulier la maladie de Tay-Sachs, de l'artériosclérose, des maladies cardiaques, de l'arthrite.

2. Solubilisat selon la revendication 1,
destiné à une utilisation comme complément alimentaire anti-inflammatoire et/ou comme médicament ayant un effet contre le cancer, la maladie d'Alzheimer, la maladie de Parkinson, l'obésité, l'hypercholestérolémie, l'hyperglycémie, le diabète, le syndrome métabolique et/ou les maladies auto-immunes, la sclérose en plaques (SEP), pour réduire la graisse viscérale, pour la thermogenèse, comme médicament pour réduire le cholestérol, en particulier le cholestérol LDL, et/ou comme médicament ayant un effet de réduction du glucose dans le sang et/ou des triglycérides dans le sang, un effet d'amélioration de la densité du pigment maculaire, un effet de réduction du stress oxydatif et/ou un effet de réduction de l'accumulation de graisse dans les hépatocytes, en particulier comme médicament ayant une action sur la stéatose hépatique, l'ataxie de Friedreich, les maladies lysomales, en particulier la maladie de Tay-Sachs, l'artériosclérose, les maladies cardiaques, l'arthrite.

3. Solubilisat contenant, en particulier composé
de curcumine dans une proportion de 3 % à 7 % en poids,
et d'au moins un autre principe actif,
qui comprend une ou plusieurs substances qui est ou sont choisies dans le groupe comprenant le xanthohumol, des extraits de plantes, notamment de résine d'arbre à encens, de réglisse, les cannabinoïdes, les enzymes, en particulier la serrapeptase, la coenzyme Q10, l'acide alpha-lipoïque, le resvératrol,
ainsi que
d'au moins un émulsifiant ayant une valeur HLB inférieure à 18, préférablement comprise entre 13 et 18, à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement entre 79 % et 88 % en poids,
destiné à une utilisation dans le domaine de l'agriculture, la pisciculture et/ou l'horticulture et/ou dans le domaine de l'hygiène alimentaire et/ou comme désinfectant et/ou dans le domaine de l'emballage, préférablement pour l'emballage de viande de boeuf, de volaille ou de poisson, et/ou comme désinfectant.

4. Solubilisat destiné à une utilisation selon la revendication 1 ou 2 ou solubilisat selon la revendication 3,
**caractérisé en ce que**
le solubilisat contient jusqu'à 20 % en poids, préférablement jusqu'à 15 % en poids d'éthanol
et/ou **en ce que**
le solubilisat contient jusqu'à 25 % en poids, préférablement entre 12 % et 20 % en poids, plus préférablement jusqu'à 10 % en poids de glycérine
et/ou **en ce que**
le solubilisat contient en outre jusqu'à 10 % en poids, préférablement jusqu'à 7 % en poids d'eau
et/ou **en ce que**
la répartition du diamètre des micelles dans une dilution du solubilisat avec de l'eau distillée dans un rapport 1:500 dans des conditions physiologiques (pH 1,1 et 37°C) va d'environ dio=6 nm à environ d9o=20 nm
et/ou **en ce que**
la turbidité du solubilisat est inférieure à 25 FNU, préférablement inférieure à 3 FNU, mesurée par diffusion de la lumière à l'aide d'une lumière infrarouge selon les prescriptions de la norme ISO 7027 pour une dilution du solubilisat dans un rapport de 1:50 ou de 1:500 dans de l'eau dans des conditions physiologiques (pH 1,1 et 37°C).

5. Capsule remplie d'un solubilisat destiné à être utilisé selon la revendication 1 ou 2 ou 4 ou d'un solubilisat selon la revendication 3 ou 4,
**caractérisée en ce que**
la capsule est conçue comme une capsule de gélatine molle ou une capsule de gélatine dure ou comme une capsule molle, exempte de gélatine, ou comme une capsule dure, exempte de gélatine, par exemple une capsule de cellulose.

6. Fluide contenant un solubilisat destiné à être utilisé selon la revendication 1 ou 2 ou 4 ou un solubilisat selon la revendication 3 ou 4,
**caractérisé en ce que**
le fluide est choisi dans le groupe comprenant les produits alimentaires, les compléments alimentaires, les boissons, les produits cosmétiques et les produits pharmaceutiques
et/ou **en ce que**
le fluide comprend une dilution aqueuse du solubilisat.

7. Solubilisat selon l'une des revendications 1 à 4 ou capsule selon la revendication 5 ou fluide selon la revendication 6, destiné à une utilisation par voie orale dans un procédé de prévention des maladies inflammatoires, du cancer, de la maladie d'Alzheimer, la maladie de Parkinson, l'obésité, l'hypercholestérolémie, l'hyperglycémie, du diabète, du syndrome métabolique et/ou des maladies auto-immunes, de la sclérose en plaques (SEP), pour réduire la graisse viscérale, pour la thermogénèse, pour réduire le cholestérol, en particulier le cholestérol LDL, et/ou le glucose dans le sang et/ou les triglycérides dans le sang, pour améliorer la densité du pigment maculaire, pour diminuer le stress oxydatif et/ou pour diminuer l'accumulation de graisse dans les hépatocytes, en particulier comme médicament destiné au traitement et/ou à la prévention de la stéatose hépatique, l'ataxie de Friedreich, des maladies lysomales, en particulier la maladie de Tay-Sachs, de l'artériosclérose, des maladies cardiaques, de l'arthrite.

8. Solubilisat destiné à une utilisation conformément à la revendication 7,
**caractérisé en ce que**
le solubilisat est administré au consommateur de complément alimentaire ou au patient à une dose de curcumine comprise entre 0,5 mg/kg de poids corporel et 1 mg/kg de poids corporel, préférablement à une dose de 0,81 mg/kg de poids corporel, en particulier une fois par jour,
et/ou **en ce que**
le solubilisat est administré au consommateur de complément alimentaire ou au patient à une dose de boswellia comprise entre 1 mg/kg de poids corporel et 2 mg/kg de poids corporel, préférablement à une dose de 1,62 mg/kg de poids corporel,
en particulier une fois par jour,
et/ou **en ce que**
le solubilisat est administré au consommateur de complément alimentaire ou au patient à une dose de xanthohumol comprise entre 0,5 mg/kg de poids corporel et 1 mg/kg de poids corporel, préférablement à une dose de 0,81 mg/kg de poids corporel.

9. Procédé de préparation d'un solubilisat contenant, en particulier composé
de curcumine dans une proportion de 3 % à 7 % en poids,
et d'au moins un autre principe actif,
qui comprend une ou plusieurs substances choisies dans le groupe comprenant le xanthohumol, des extraits de plantes, notamment de résine d'arbre à encens, de réglisse, les cannabinoïdes, les enzymes, en particulier la serrapeptase, la coenzyme Q10, l'acide alpha-lipoïque, le resvératrol,
ainsi que
d'au moins un émulsifiant ayant une valeur HLB inférieure à 18, préférablement comprise entre 13 et 18,
à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement comprise entre 79 % et 88 % en poids, en particulier d'un solubilisat selon la revendication 4,
comprenant les étapes suivantes
a) introduction de polysorbate 80 et/ou de polysorbate 20
et/ou d'un mélange de polysorbate 20 et de polysorbate 80
b) ajout d'au moins un autre principe actif, en particulier d'un extrait de *Boswellia serrata* et/ou de
xanthohumol,
c) ajout de poudre de curcumine
à l'étape a), un chauffage à une température comprise entre 40°C et 62°C, préférablement à une température comprise entre 45°C à 57°C, plus préférablement à une température comprise entre 48°C et 52°C, étant effectué et
à l'étape b), la température restant inchangée par rapport à l'étape a) ou un chauffage à une température comprise entre 60°C et 75°C, préférablement à une température comprise entre 61°C et 70°C, plus préférablement à une température comprise entre 63°C et 67°C, étant effectué, et
à l'étape c), un chauffage à une température comprise entre 82°C et 97°C, préférablement à une température
comprise entre 83°C et 92°C, plus préférablement à une température comprise entre 85°C et 89°C, étant effectué.

10. Procédé selon la revendication 9,
avant l'étape b), une étape
bl) ajout d'eau à une température comprise entre 40°C et 62°C, préférablement à une température comprise entre 45°C et 57°C, plus préférablement à une température comprise entre 48°C et 52°C, étant effectuée.

11. Procédé selon la revendication 9 ou 10,
à l'étape
b1), également l'ajout d'éthanol à une température comprise entre 40°C et 62°C, préférablement à une température comprise entre 45°C à 57°C, plus préférablement à une température comprise entre 48°C et 52°C,
étant effectué.

12. Procédé de préparation d'un solubilisat contenant, en particulier composé
de curcumine dans une proportion de 3 % à 7 % en poids,
et d'au moins un autre principe actif,
qui comprend une ou plusieurs substances choisies dans le groupe comprenant le xanthohumol, des extraits de plantes, notamment de résine d'arbre à encens, de réglisse, les cannabinoïdes, les enzymes, en particulier la serrapeptase, la coenzyme 010, l'acide alpha-lipoïque, le resvératrol,
ainsi que
d'au moins un émulsifiant ayant une valeur HLB inférieure à 18, préférablement comprise entre 13 et 18,
à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement comprise entre 79 % et 88 % en poids, en particulier d'un solubilisat selon la revendication 4
comprenant les étapes suivantes
a) réalisation d'une première préparation par l'introduction de polysorbate 80 et/ou de polysorbate 20 et/ou d'un mélange de polysorbate 20 et de polysorbate 80 et d'au moins un premier principe actif, en particulier l'acide alpha-lipoïque,
b) réalisation d'une deuxième préparation par l'introduction d'eau et d'au moins un autre principe actif, en particulier la serrapeptase
c) ajout d'au moins un autre principe actif, en particulier de xanthohumol et/ou d'extrait de boswellia serrata et/ou de glavonoïde et/ou de resvératrol et/ou de coenzyme Q10, et ajout de poudre de curcumine à la deuxième préparation de l'étape b)
d) combinaison de la première préparation de l'étape a) et de la deuxième préparation de l'étape c), la température étant comprise entre 18°C et 22°C pendant la réalisation des étapes a) à d),
e) chauffage à une température comprise entre 80°C et 97°C, préférablement à une température comprise entre 83°C et 92°C, plus préférablement à une température comprise entre 85°C et 89°C.

13. Procédé selon la revendication 12,
à l'étape
c), également l'ajout d'éthanol et/ou de glycérol et/ou
d'huile MCT et/ou de polysorbate 20 et/ou de polysorbate 80 et/ou d'un mélange de polysorbate 20 et de polysorbate 80 étant effectué.

14. Procédé de préparation d'un solubilisat contenant, en particulier composé
de curcumine dans une proportion de 3 % à 7 % en poids,
et d'au moins un autre principe actif,
qui comprend une ou plusieurs substances qui est ou sont choisies dans le groupe comprenant le xanthohumol, des extraits de plantes, notamment de résine d'arbre à encens, de réglisse, les cannabinoïdes, les enzymes, en particulier la serrapeptase, la coenzyme 010, l'acide alpha-lipoïque, le resvératrol,
ainsi que
d'au moins un émulsifiant ayant une valeur HLB inférieure à 18, préférablement comprise entre 13 et 18,
à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement comprise entre 79 % et 88 % en poids, en particulier d'un solubilisat selon la revendication 4
comprenant les étapes suivantes
a) introduction de polysorbate 80 et/ou de polysorbate 20 et/ou d'un mélange de polysorbate 20 et de polysorbate 80 et de glycérol et d'éthanol,
b) ajout d'au moins un autre principe actif, en particulier d'un extrait éthanolique des résines dures de houblon,
à l'étape a), un chauffage à une température comprise entre 40°C et 62°C, préférablement à une température comprise entre 45°C et 57°C, plus préférablement à une température comprise entre 48°C et 52°C, étant effectué
et
à l'étape b), un chauffage à une température comprise entre 60°C et 75°C, préférablement à une température comprise entre 61°C et 70°C, plus préférablement à une température comprise entre 63°C et 67°C, étant effectué,
c) ajout de poudre de curcumine à une température comprise entre 70°C et 92°C, préférablement à une température comprise entre 75°C et 87°C, plus préférablement à une température comprise entre 78°C et 82°C,
d) ajout de glavonoïdes par chauffage à une température comprise entre 80°C et 97°C, préférablement à une température comprise entre 83°C et 92°C, plus préférablement à une température comprise entre 85°C et 89°C.

15. Procédé de préparation d'un solubilisat contenant, en particulier composé
de curcumine dans une proportion de 3 % à 7 % en poids,
et d'au moins un autre principe actif,
qui comprend une ou plusieurs substances qui est ou sont choisies dans le groupe comprenant le xanthohumol, des extraits de plantes, notamment de résine d'arbre à encens, de réglisse, les cannabinoïdes, les enzymes, en particulier la serrapeptase, la coenzyme 010, l'acide alpha-lipoïque, le resvératrol,
ainsi que
d'au moins un émulsifiant ayant une valeur HLB inférieure à 18, préférablement comprise entre 13 et 18,
à savoir le polysorbate 80 ou le polysorbate 20 ou un mélange de polysorbate 20 et de polysorbate 80,
la proportion de polysorbate étant d'au moins 70 % en poids, préférablement comprise entre 75 % et 95 % en poids, plus préférablement comprise entre 79 % et 88 % en poids, en particulier d'un solubilisat selon la revendication 4,
par le mélange d'un solubilisat de curcumine et d'au moins un autre solubilisat de principe actif, en particulier dans un rapport quantitatif 1:1 des différents solubilisats.
